# EUROPÄISCHE PATENTSCHRIFT

(11) **EP 4 334 733 B1**
(45) Veröffentlichungstag und Bekanntmachung des Hinweises auf die Patenterteilung: **21.01.2026**
(21) Anmeldenummer: 22724112.2
(22) Anmeldetag: 28.04.2022
(51) Int. Cl.: G01R 33/56, A61B 5/055, G06T 7/00, A61K 49/10

(54) **CHARAKTERISIERUNG VON LÄSIONEN IN DER LEBER MITTELS DYNAMISCHER KONTRASTVERSTÄRKTER MAGNETRESONANZTOMOGRAPHIE**
CHARACTERIZATION OF LESIONS IN THE LIVER WITH DYNAMIC CONTRAST-ENHANCED MAGNETIC RESONANCE IMAGING
CARACTÉRISATION DES LÉSIONS DANS LE FOIE PAR RÉSONANCE MAGNÉTIQUE DYNAMIQUE À CONTRASTE AMÉLIORÉ

(30) Priorität: 07.05.2021 EP 21172677
(43) Veröffentlichungstag der Anmeldung: 13.03.2024
(73) Patentinhaber: Bayer Aktiengesellschaft, 51373 Leverkusen (DE)
(72) Erfinder: SCHUETZ, Gunnar, 13467 Berlin (DE); KNOBLOCH, Gesine, 13507 Berlin (DE)
(74) Vertreter: BIP Patents
(86) Internationale Anmeldenummer: PCT/EP2022/061289
(87) Internationale Veröffentlichungsnummer: WO 2022/233689

(56) Entgegenhaltungen:
- US-A1- 2012 070 055
- EUROPEAN ASSOCIATION FOR THE STUDY OF THE LIVER (EASL): "EASL Clinical Practice Guidelines: Management of hepatocellular carcinoma", JOURNAL OF HEPATOLOGY, ELSEVIER, AMSTERDAM, NL, vol. 69, no. 1, 5 April 2018 (2018-04-05), pages 182 - 236, XP085407560, ISSN: 0168-8278, DOI: 10.1016/J.JHEP.2018.03.019
- ZECH CHRISTOPH J ET AL: "Consensus report from the 8th International Forum for Liver Magnetic Resonance Imaging", EUROPEAN RADIOLOGY, SPRINGER INTERNATIONAL, BERLIN, DE, vol. 30, no. 1, 5 August 2019 (2019-08-05), pages 370 - 382, XP037008833, ISSN: 0938-7994, [retrieved on 20190805], DOI: 10.1007/S00330-019-06369-4

## Beschreibung

Die vorliegende Erfindung befasst sich mit dem technischen Gebiet der Charakterisierung von Läsionen in der Leber mittels dynamischer kontrastverstärkter Magnetresonanztomographie.

Die Leber kann von mehreren gutartigen Tumoren betroffen sein, die als zystische oder feste fokale Läsionen im Leberparenchym auftreten können. Die Leber ist jedoch auch anfällig für bösartige Tumoren wie Metastasen von extrahepatischen Krebsarten oder von primären Krebsarten, die ihren Ursprung in der Leber selbst haben. Weltweit sind die beiden häufigsten Arten von bösartigen Lebertumoren Metastasen - insbesondere Darmkrebs-Metastasen - und hepatozelluläre Karzinome (HCC). Fast 20% der Patienten mit Darmkrebs haben zum Zeitpunkt der Diagnose Lebermetastasen, und mehr als 50% der Patienten mit Darmkrebs entwickeln im Verlauf ihrer Krankheit Lebermetastasen. Das hepatozelluläre Karzinom (HCC) ist der häufigste primäre Leberkrebs. Es ist auch die sechsthäufigste Krebserkrankung weltweit und die vierthäufigste Ursache für krebsbedingte Todesfälle.

Die genaue und zuverlässige Erkennung und Charakterisierung von fokalen Leberläsionen in frühen Krankheitsstadien ist von hoher klinischer Relevanz, insbesondere bei Patienten mit einem Risiko für Lebermetastasen oder primären Leberkrebs, da sie für eine angemessene Behandlungsplanung von grundlegender Bedeutung sind und die Eignung für potenziell kurative Behandlungsoptionen bestimmen.

Die Magnetresonanztomographie (MRT) ist von besonderer Bedeutung für die radiologische Untersuchung von Leberläsionen. Sie zeichnet sich durch einen hervorragenden Weichteilkontrast und eine hohe räumliche Auflösung aus, ohne den Patienten ionisierender Strahlung oder iodierten Kontrastmitteln auszusetzen.

Die in der MRT am häufigsten verwendeten Kontrastmittel sind paramagnetische Kontrastmittel auf Gadolinium-Basis. Diese Mittel werden über eine intravenöse (i.v.) Bolusinjektion verabreicht. Ihre kontrastverstärkende Wirkung wird durch das zentrale Gadolinium-Ion (Gd - III) im Chelatkomplex vermittelt. Wenn T1-gewichtete (w) Abtastsequenzen in der MRT verwendet werden, führt die durch Gadolinium-Ionen induzierte Verkürzung der Spin-Gitter-Relaxationszeit (T1) von angeregten Atomkernen zu einer Erhöhung der Signalintensität und damit zu einer Erhöhung des Bildkontrasts des untersuchten Gewebes.

Nach ihrem Verteilungsmuster im Gewebe können Gadolinium-basierte Kontrastmittel grob in extrazelluläre und intrazelluläre Kontrastmittel unterteilt werden.

Als extrazelluläre Kontrastmittel werden niedermolekulare, wasserlösliche Verbindungen bezeichnet, die sich nach intravenöser Gabe in den Blutgefäßen und im interstitiellen Raum verteilen. Sie werden nach einer gewissen, vergleichsweise kurzen Zeitspanne der Zirkulation im Blutkreislauf über die Nieren ausgeschieden. Zu den extrazellulären MRT-Kontrastmitteln zählen beispielsweise die Gadolinium-Chelate Gadobutrol (Gadovist^{®}), Gadoteridol (Prohance^{®}), Gadotersäure (Dotarem^{®}), Gadopentetsäure (Magnevist^{®}) und Gadodiamid (Omnican^{®}).

Intrazelluläre Kontrastmittel werden zu gewissen Anteilen in die Zellen von Geweben aufgenommen und anschließend wieder ausgeschieden. Intrazelluläre MRT-Kontrastmittel auf der Basis der Gadoxetsäure zeichnen sich beispielsweise dadurch aus, dass sie anteilig spezifisch von Leberzellen, den Hepatozyten, aufgenommen werden, sich im Funktionsgewebe (Parenchym) anreichern und die Kontraste in gesundem Lebergewebe verstärken, bevor sie anschließend über Galle in die Faeces ausgeschieden werden. Beispiele für derartige Kontrastmittel auf der Basis der Gadoxetsäure sind in US 6,039,931A beschrieben; sie sind kommerziell zum Beispiel unter den Markennamen Primovist^{®} und Eovist^{®} erhältlich. Ein weiteres MRT-Kontrastmittel mit einer geringeren Aufnahme in die Hepatozyten ist Gadobenate Dimeglumine (Multihance^{®}).

Gadoxetat-Dinatrium (GD, Primovist^{®}) gehört zur Gruppe der intrazellulären Kontrastmittel. Es ist zur Verwendung in der MRT der Leber zugelassen, um Läsionen bei Patienten mit bekannter oder vermuteter fokaler Lebererkrankung zu erkennen und zu charakterisieren. GD weist mit seiner lipophilen Ethoxybenzyl-Einheit eine zweiphasige Verteilung auf: zunächst eine Verteilung im intravaskulären und interstitiellen Raum nach Bolusinjektion, gefolgt von einer selektiven Aufnahme durch Hepatozyten. GD wird über die Nieren und den hepatobiliären Weg (50:50 dualer Ausscheidungsmechanismus) in etwa gleichen Mengen unverändert aus dem Körper ausgeschieden. GD wird aufgrund seiner selektiven Anreicherung im gesunden Lebergewebe auch als hepatobiliäres Kontrastmittel bezeichnet.

GD ist in einer Dosis von 0,1 ml / kg Körpergewicht (BW) (0,025 mmol / kg BW Gd) zugelassen. Die empfohlene Verabreichung von GD umfasst eine unverdünnte intravenöse Bolusinjektion mit einer Flussrate von ungefähr 2 ml / Sekunde, gefolgt von einer Spülung der i.v. Kanüle mit einer physiologischen Kochsalzlösung. Ein Standardprotokoll für die Leberbildgebung unter Verwendung von GD besteht aus mehreren Planungs- und Vorkontrastsequenzen. Nach i.v. Bolusinjektion des Kontrastmittels, werden üblicherweise dynamische Aufnahmen während der arteriellen (ungefähr 30 Sekunden nach der Injektion, p.i.), portalvenösen (ungefähr 60 Sekunden p.i.) und der Übergangsphase (ungefähr 2-5 Minuten p.i.) aufgenommen. Die Übergangsphase zeigt typischerweise bereits einen gewissen Anstieg der Lebersignalintensität aufgrund der beginnenden Aufnahme des Mittels in Hepatozyten. Zusätzliche T2-gewichtete und diffusionsgewichtete (DWI) Aufnahmen können nach der dynamischen Phase und vor der späten hepatobiliären Phase erzeugt werden.

Die kontrastverstärkten dynamischen Aufnahmen aus der arteriellen, portalvenösen und der Übergangsphase liefern entscheidende Informationen über die zeitlich variierenden Muster der Läsionsverstärkung (Vaskularisation), die zur Charakterisierung der spezifischen Leberläsion beitragen. Das hepatozelluläre Karzinom, mit seinem typischen arteriellen Phasenhyperenhancement (APHE) und dem Auswaschen (engl.: *washout*) des Kontrastes in der venösen Phase, kann allein anhand seines einzigartigen Vaskularisierungsmuster diagnostiziert werden, das während der dynamischen Phasenbildgebung beobachtet wird, wodurch Patienten vor einer invasiven und möglicherweise riskanten Leberbiopsie geschützt werden.

Auch andere Läsionen lassen sich mittels dynamischer kontrastverstärkter MRT charakterisieren.

In der Diagnose von Leberläsionen hat ein hepatobiliäres Kontrastmittel gegenüber einem extrazellulären Kontrastmittel den Vorteil, dass es eine höhere Sensitivität aufweist und damit insbesondere kleinere Karzinome besser erkennen lässt (siehe z.B.: R.F. Hanna et al.: Comparative 13-year meta-analysis of the sensitivity and positive predictive value of ultrasound, CT, and MRI for detecting hepatocellular carcinoma, Abdom Radiol 2016, 41, 71-90; Y.J. Lee et al.: Hepatocellular carcinoma: diagnostic performance ofmultidetector CT and MR imaging-a systematic review and meta-analysis, Radiology 2015, 275, 97-109; D.K Owens et al.: High-value, cost-conscious health care: concepts for clinicians to evaluate the benefits, harms, and costs of medical interventions, Ann Intern Med 2011, 154, 174-180).

Das Problem besteht nun darin, dass die dynamische Kontrastzunahme und/oder -abnahme in Leberläsionen von Radiologen häufig per Augenschein beurteilt wird (subjektive Beurteilung der relativen Kontraständerung zwischen Lebergewebe und Leberläsion) und dabei Fehlinterpretationen auftreten können.

Bei einer augenscheinlichen Beurteilung macht es einen Unterschied, ob ein extrazelluläres oder ein intrazelluläres Kontrastmittel verwendet wird. Bei Verwendung eines hepatobiliären Kontrastmittels kann beispielsweise eine Signalzunahme in gesundem Lebergewebe als eine Auswaschung von Kontrastmittel aus benachbarten Leberläsionen fehlinterpretiert werden (relative Kontrastzunahme zwischen Lebergewebe und Leberläsion).

Daher hat die *European Association for the Study of Liver* in ihren Richtlinien (*EASL Clinical Practice Guidelines: Management of hepatocellular carconima*), in Abhängigkeit des verwendeten Kontrastmittels unterschiedliche Merkmale für die Identifizierung eines hepatozellulären Karzinoms angegeben: (siehe Journal of Hepatology, 2018, Vol. 69, Seiten 182-236):
- bei Verwendung eines extrazellulären Kontrastmittels: Kombination aus Hypervaskularität in der späten arteriellen Phase und Auswaschen in der portalvenösen und/oder der verzögerten Phase,
- bei Verwendung eines hepatobiliären Kontrastmittels: Kombination aus Hypervaskularität in der späten arteriellen Phase und Auswaschen in der portalvenösen Phase.

Während also bei der Verwendung eines extrazellulären Kontrastmittels zur Erkennung von hepatozellulären Karzinomen sowohl die portalvenöse als auch die anschließende verzögerte Phase herangezogen werden, wird bei Verwendung eines hepatobiliären Kontrastmittel nur die portalvenöse Phase zur Erkennung einer Auswaschung herangezogen und keine an die portalvenöse Phase anschließende Phase.

Der Grund hierfür ist, wie bereits beschrieben, dass ein hepatobiliäres Kontrastmittel (im Gegensatz zu einem extrazellulären Kontrastmittel) von den Leberzellen aufgenommen wird und sich dort anreichert, bevor es über Galle in die Faeces ausgeschieden wird. Es kommt also bei der Verwendung eines hepatobiliären Kontrastmittels im Anschluss an eine erste vergleichsweise schnelle Gefäß-Kontrastverstärkung in der arteriellen Phase, zu einer langsam kontinuierlich weiter zunehmenden Kontrastverstärkung in gesundem Lebergewebe. Vergleicht ein Radiologe visuell die Kontrastverstärkung in einer Läsion mit der Kontrastverstärkung in gesundem Lebergewebe, kann die kontinuierlich zunehmende Kontrastverstärkung in gesundem Lebergewebe als eine Auswaschung von Kontrastmittel aus Läsionen fehlinterpretiert werden.

Die *European Association for the Study of Liver* empfiehlt also explizit, bei der Verwendung eines hepatobiliäten Kontrastmittels, die Analyse der MRT-Aufnahmen zur Identifizierung eines hepatozellulären Karzinoms auf die arterielle und die portalvenöse zu beschränken (bis 60 Sekunden nach der intravenösen Applikation des Kontrastmittels).

Das Problem liegt aber darin, dass sich manche Läsionen erst durch das dynamische Verhalten in einer späteren Phase eindeutig charakterisieren lassen.

Bei manchen hepatozellulären Karzinomen zeigt sich die Auswaschung beispielsweise erst nach der portalvenösen Phase (siehe z.B. C. J. Zech et al.: Consensus report from the 8th International Forum for Liver Magnetic Resonance Imaging, European Radiology 2020, 30, 370-382).

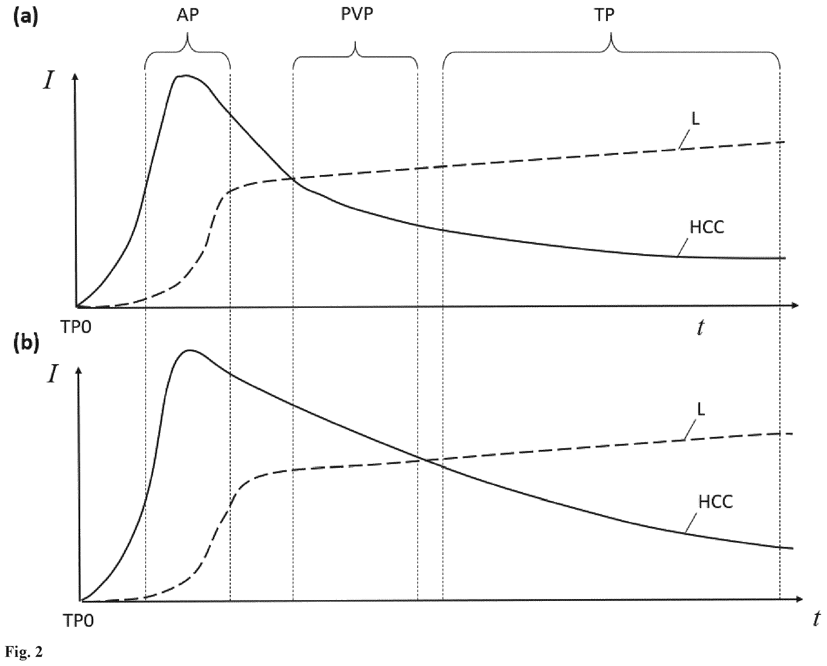

Wird die Zeitspanne nach der portalvenösen Phase, wie von der *European Association for the Study of Liver* empfohlen, nicht für die Erkennung einer Auswaschung berücksichtigt, so bleiben beispielsweise solche hepatozellulären Karzinome, bei denen die Auswaschung erst nach der portalvenösen Phase erkennbar wird, unerkannt. Die Folge kann sein, dass mehr Biopsien durchgeführt werden müssen, um zu prüfen, ob es sich bei einer Läsion um eine gutartige Läsion oder um einen bösartigen Tumor handelt (siehe insbesondere Fig. 2 in Journal of Hepatology, 2018, Vol. 69, Seite 194). Eine Biopsie ist nicht nur ein zusätzlicher Aufwand für das medizinische Personal; sie bedeutet für den Patienten auch ein Risiko.

Es wäre wünschenswert, die Zahl von solchen Biopsien reduzieren zu können. Es wäre wünschenswert, Leberläsionen zuverlässig identifizieren und charakterisieren zu können, ohne dass die Gefahr besteht, dass bei Verwendung eines hepatobiliären Kontrastmittels ein Signalanstieg von gesundem Lebergewebe als ein Auswaschen von Kontrastmittel aus Leberläsionen fehlinterpretiert wird.

Dies wird durch die vorliegende Erfindung erreicht.

Ein erster Gegenstand der vorliegenden Erfindung ist ein computer-implementiertes Verfahren zur Identifizierung einer Auswaschung von Kontrastmittel aus einem Bereich einer Leber eines Patienten während einer dynamischen kontrastverstärkten magnetresonanztomographischen Untersuchung, umfassend die Schritte:
- Empfangen einer Mehrzahl an Repräsentationen,
   ∘ wobei die Mehrzahl an Repräsentationen
      ▪ die Leber des Patienten oder einen Teil der Leber des Patienten, und
      ▪ Referenzgewebe des Patienten
      repräsentiert,
   ∘ wobei mindestens eine Repräsentation der Mehrzahl an Repräsentationen
      ▪ die Leber oder den Teil der Leber, und
      ▪ das Referenzgewebe
      während einer portalvenösen Phase der dynamischen kontrastverstärkten magnetresonanztomographischen Untersuchung repräsentiert, und
   ∘ wobei mindestens eine Repräsentation der Mehrzahl an Repräsentationen
      ▪ die Leber oder den Teil der Leber, und
      ▪ das Referenzgewebe
      während einer Übergangsphase der dynamischen kontrastverstärkten magnetresonanztomographischen Untersuchung repräsentiert,
   wobei es sich bei dem Kontrastmittel um ein hepatobiliäres Kontrastmittel handelt, wobei es sich bei dem Referenzgewebe um Muskelgewebe handelt,
- Analysieren der Repräsentationen und dabei Identifizieren eines oder mehrerer Bereiche in der Leber,
   ∘ bei dem/denen Kontrastmittel in der portalvenösen Phase und/oder der Übergangsphase zu einer geringeren Kontrastverstärkung als in dem Referenzgewebe führt, und/oder
   ∘ bei dem/denen die Kontrastverstärkung in der portalvenösen Phase und/oder der Übergangsphase schneller sinkt als in dem Referenzgewebe, wobei das Referenzgewebe keine Hepatozyten umfasst, und/oder
   ∘ bei dem/denen der Betrag des Gradienten der abnehmenden Kontrastverstärkung in der portalvenösen Phase und/oder der Übergangsphase größer ist als der Betrag des Gradienten der zunehmenden Kontrastverstärkung in gesundem Lebergewebe,
- Ausgeben einer Repräsentation der Leber oder des Teils der Leber, wobei in der Repräsentation der identifizierte Bereich hervorgehoben ist oder die identifizierten Bereiche hervorgehoben sind.

Ein weiterer Gegenstand der vorliegenden Erfindung ist ein Computersystem umfassend
- eine Empfangseinheit,
- eine Steuer- und Recheneinheit und
- eine Ausgabeeinheit,
   - wobei die Steuer- und Recheneinheit konfiguriert ist, die Empfangseinheit zu veranlassen, eine Mehrzahl an Repräsentationen zu empfangen,
      ∘ wobei die Mehrzahl an Repräsentationen
         ▪ eine Leber eines Patienten oder einen Teil der Leber des Patienten, und
         ▪ Referenzgewebe des Patienten
         repräsentiert,
      ∘ wobei mindestens eine Repräsentation der Mehrzahl an Repräsentationen
         ▪ die Leber oder den Teil der Leber, und
         ▪ das Referenzgewebe
         während einer portalvenösen Phase einer dynamischen kontrastverstärkten magnetresonanztomographischen Untersuchung repräsentiert, und
      ∘ wobei mindestens eine Repräsentation der Mehrzahl an Repräsentationen
         ▪ die Leber oder den Teil der Leber, und
         ▪ das Referenzgewebe
         während einer Übergangsphase der dynamischen kontrastverstärkten magnetresonanztomographischen Untersuchung repräsentiert,
      wobei es sich bei dem Kontrastmittel um ein hepatobiliäres Kontrastmittel handelt, wobei es sich bei dem Referenzgewebe um Muskelgewebe handelt,
   - wobei die Steuer- und Recheneinheit konfiguriert ist, die Repräsentationen zu analysieren und dabei einen oder mehrere Bereiche in der Leber zu identifizieren,
      ∘ bei dem/denen Kontrastmittel in der portalvenösen Phase und/oder der Übergangsphase zu einer geringeren Kontrastverstärkung als in dem Referenzgewebe führt, und/oder
      ∘ bei dem/denen die Kontrastverstärkung in der portalvenösen Phase und/oder der Übergangsphase schneller sinkt als in dem Referenzgewebe, wobei das Referenzgewebe keine Hepatozyten umfasst, und/oder
      ∘ bei dem/denen der Betrag des Gradienten der abnehmenden Kontrastverstärkung in der portalvenösen Phase und/oder der Übergangsphase größer ist als der Betrag des Gradienten der zunehmenden Kontrastverstärkung in gesundem Lebergewebe,
   - wobei die Steuer- und Recheneinheit konfiguriert ist, die Ausgabeeinheit zu veranlassen, eine Repräsentation der Leber oder des Teils der Leber auszugeben, wobei in der Repräsentation der identifizierte Bereich hervorgehoben ist oder die identifizierten Bereiche hervorgehoben sind.

Ein weiterer Gegenstand der vorliegenden Erfindung ist ein Computerprogrammprodukt umfassend ein Computerprogramm, das in einen Arbeitsspeicher eines Computers geladen werden kann und dort den Computer dazu veranlasst, folgende Schritte ausführen:
- Empfangen einer Mehrzahl an Repräsentationen,
   ∘ wobei die Mehrzahl an Repräsentationen
      ▪ eine Leber eines Patienten oder einen Teil der Leber des Patienten, und
      ▪ Referenzgewebe des Patienten
      repräsentiert,
   ∘ wobei mindestens eine Repräsentation der Mehrzahl an Repräsentationen
      ▪ die Leber oder den Teil der Leber, und
      ▪ das Referenzgewebe
      während einer portalvenösen Phase einer dynamischen kontrastverstärkten magnetresonanztomographischen Untersuchung repräsentiert, und
   ∘ wobei mindestens eine Repräsentation der Mehrzahl an Repräsentationen
      ▪ die Leber oder den Teil der Leber, und
      ▪ das Referenzgewebe
      während einer Übergangsphase der dynamischen kontrastverstärkten magnetresonanztomographischen Untersuchung repräsentiert,
   wobei es sich bei dem Kontrastmittel um ein hepatobiliäres Kontrastmittel handelt, wobei es sich bei dem Referenzgewebe um Muskelgewebe handelt,
- Analysieren der Repräsentationen und dabei Identifizieren eines oder mehrerer Bereiche in der Leber,
   ∘ bei dem/denen Kontrastmittel in der portalvenösen Phase und/oder der Übergangsphase zu einer geringeren Kontrastverstärkung als in dem Referenzgewebe führt, und/oder
   ∘ bei dem/denen die Kontrastverstärkung in der portalvenösen Phase und/oder der Übergangsphase schneller sinkt als in dem Referenzgewebe, wobei das Referenzgewebe keine Hepatozyten umfasst, und/oder
   ∘bei dem/denen der Betrag des Gradienten der abnehmenden Kontrastverstärkung in der portalvenösen Phase und/oder der Übergangsphase größer ist als der Betrag des Gradienten der zunehmenden Kontrastverstärkung in gesundem Lebergewebe,
- Ausgeben einer Repräsentation der Leber oder des Teils der Leber, wobei in der Repräsentation der identifizierte Bereich hervorgehoben ist oder die identifizierten Bereiche hervorgehoben sind.

Ein weiterer Gegenstand der vorliegenden Erfindung ist die Verwendung eines hepatobiliären Kontrastmittels in einem dynamischen magnetresonanztomographischen Untersuchungsverfahren, wobei das Untersuchungsverfahren die folgenden Schritte umfasst:
- Applizieren des Kontrastmittels in eine von einer Arterie entfernte Vene eines Patienten,
- Erzeugen einer Mehrzahl an Repräsentationen,
   ∘ wobei die Mehrzahl an Repräsentationen
      ▪ eine Leber eines Patienten oder einen Teil der Leber des Patienten, und
      ▪ Referenzgewebe des Patienten
      repräsentiert,
   ∘ wobei mindestens eine Repräsentation der Mehrzahl an Repräsentationen
      ▪ die Leber oder den Teil der Leber, und
      ▪ das Referenzgewebe
      während einer portalvenösen Phase des Untersuchungsverfahrens repräsentiert, und
   ∘ wobei mindestens eine Repräsentation der Mehrzahl an Repräsentationen
      ▪ die Leber oder den Teil der Leber, und
      ▪ das Referenzgewebe
      während einer Übergangsphase des Untersuchungsverfahrens repräsentiert,
   wobei es sich bei dem Referenzgewebe um Muskelgewebe handelt,
- Analysieren der Repräsentationen und dabei Identifizieren eines oder mehrerer Bereiche in der Leber,
   ∘ bei dem/denen Kontrastmittel in der portalvenösen Phase und/oder der Übergangsphase zu einer geringeren Kontrastverstärkung als in dem Referenzgewebe führt, und/oder
   ∘ bei dem/denen die Kontrastverstärkung in der portalvenösen Phase und/oder der Übergangsphase schneller sinkt als in dem Referenzgewebe, wobei das Referenzgewebe keine Hepatozyten umfasst, und/oder
   ∘ bei dem/denen der Betrag des Gradienten der abnehmenden Kontrastverstärkung in der portalvenösen Phase und/oder der Übergangsphase größer ist als der Betrag des Gradienten der zunehmenden Kontrastverstärkung in gesundem Lebergewebe,
- Ausgeben einer Repräsentation der Leber oder des Teils der Leber, wobei in der Repräsentation der identifizierte Bereich hervorgehoben ist oder die identifizierten Bereiche hervorgehoben sind.

Ein weiterer Gegenstand der vorliegenden Erfindung ist ein Kit umfassend ein Kontrastmittel und das erfindungsgemäße Computerprogrammprodukt.

Bevorzugte Ausführungsformen der Erfindung finden sich in den abhängigen Patentansprüchen, der vorliegenden Beschreibung sowie in den Zeichnungen.

Die Erfindung wird nachstehend näher erläutert, ohne zwischen den Erfindungsgegenständen (Verfahren, Computersystem, Computerprogrammprodukt, Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, Computersystem, Computerprogrammprodukt, Verwendung, Kit) sie erfolgen.

Die vorliegende Erfindung stellt Mittel zur automatisierten Identifizierung einer Auswaschung (engl.: *washout*) von Kontrastmittel aus einem Bereich einer Leber eines Patienten während einer dynamischen kontrastverstärkten magnetresonanztomografischen Untersuchung bereit. Mit anderen Worten: mit Hilfe der vorliegenden Erfindung kann ein Bereich oder es können mehrere Bereiche innerhalb der Leber automatisch identifiziert werden, die durch ein Auswaschen von Kontrastmittel charakterisiert sind.

Als Auswaschen/Auswaschung (engl.: *washout*) wird die Beobachtung bezeichnet, dass die Kontrastverstärkung in einem Bereich einer Leber in der portalvenösen Phase und/oder der Übergangsphase einer dynamischen kontrastverstärkten magnetresonanztomografischen Untersuchung schneller absinkt als im umgebenden (gesunden) Lebergewebe.

Eine solche Auswaschung wird oft als ein charakteristisches Merkmal zum Spezifizieren von Leberläsionen herangezogen (siehe z.B.: Y. I. Liu et al.: Quantitatively Defining Washout in Hepatocellular Carcinoma, American Journal of Roentgenology 2013 200:1, 84-89; Journal of Hepatology, 2018, Vol. 69, Seiten 182-236).

Die Magnetresonanztomographie, abgekürzt MRT (engl. MRI: *Magnetic Resonance Imaging*)*,* ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MRT-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer Hochfrequenz-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

In einem ersten Schritt einer dynamischen kontrastverstärkten magnetresonanztomografischen Untersuchung wird einem Untersuchungsobjekt ein MRT-Kontrastmittel verabreicht.

Das "Untersuchungsobjekt" ist üblicherweise ein Lebewesen, vorzugsweise ein Säugetier, ganz besonders bevorzugt ein Mensch. In dieser Beschreibung wird auch der Begriff Patient verwendet.

Das Kontrastmittel ist erfindungsgemäß ein hepatobiliäres Kontrastmittel.

Unter einem hepatobiliären Kontrastmittel wird ein Kontrastmittel verstanden, das spezifisch von gesunden Leberzellen, den Hepatozyten, aufgenommen wird.

Bespiele für hepatobiliäre Kontrastmittel sind Kontrastmittel auf der Basis der Gadoxetsäure. Sie sind beispielsweise in US 6,039,931A beschrieben. Sie sind kommerziell zum Beispiel unter den Markennamen Primovist^{®} oder Eovist^{®} erhältlich

Der kontrastverstärkende Effekt von Primovist^{®}/Eovist^{®} wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels.

In einer besonders bevorzugten Ausführungsform handelt es sich bei dem verwendeten Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

Nach der intravenösen Applikation des hepatobiliären Kontrastmittels in Form eines Bolus in eine Armvene erreicht das Kontrastmittel die Leber zunächst über die Arterien. Diese sind in den entsprechenden MRT-Aufnahmen kontrastverstärkt dargestellt. Die Phase, in der die Leberarterien in MRT-Aufnahmen kontrastverstärkt dargestellt sind, wird als "arterielle Phase" bezeichnet.

Anschließend erreicht das Kontrastmittel die Leber über die Lebervenen. Während der Kontrast in den Leberarterien bereits abnimmt, erreicht der Kontrast in den Lebervenen ein Maximum. Die Phase, in der die Lebervenen in MRT-Aufnahmen kontrastverstärkt dargestellt sind, wird als "portalvenöse Phase" bezeichnet. Diese Phase kann bereits während der arteriellen Phase beginnen und sich mit dieser überlagern.

An die portalvenöse Phase schließt sich die "Übergangsphase" (engl. *transitional phase*) an, in der der Kontrast in den Leberarterien weiter absinkt, der Kontrast in den Lebervenen ebenfalls absinkt. Bei Verwendung eines hepatobiliären Kontrastmittels steigt der Kontrast in den gesunden Leberzellen in der Übergangsphase allmählich an.

Die arterielle Phase, die portalvenöse Phase und die Übergangsphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

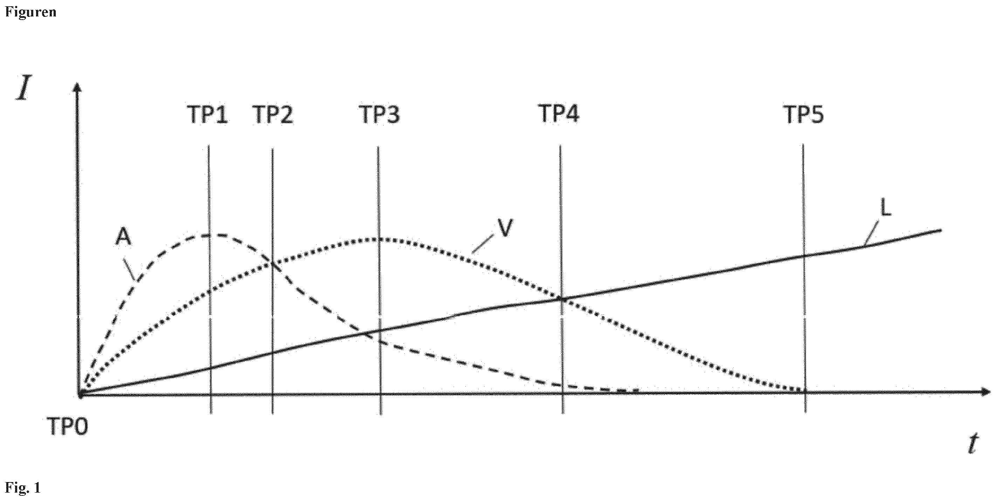

Figur 1 zeigt schematisch den zeitlichen Verlauf (*t* = Zeit) der Signalintensitäten *I,* die durch ein hepatobiliäres Kontrastmittel in Leberarterien (A), Lebervenen (V) und gesunden Leberzellen (L) bei einer dynamischen kontrastverstärkten MRT-Untersuchung hervorgerufen werden. Die Signalintensität *I* korreliert positiv mit der Konzentration des Kontrastmittels in den genannten Bereichen. Bei einer intravenösen Bolusinjektion steigt die Konzentration des Kontrastmittels in den Leberarterien (A) als erstes an (gestrichelte Kurve). Die Konzentration durchläuft ein Maximum und sinkt dann ab. Die Konzentration in den Lebervenen (V) steigt langsamer an als in den Leberarterien und erreicht ihr Maximum später (gepunktete Kurve). Die Konzentration des Kontrastmittels in den Leberzellen (L) steigt langsam an (durchgezogene Kurve) und erreicht ihr Maximum erst zu einem sehr viel späteren Zeitpunkt (in der Figur 1 nicht dargestellt). Es lassen sich einige charakteristische Zeitpunkte definieren: Zum Zeitpunkt TP0 wird Kontrastmittel intravenös als Bolus appliziert. Da die Applikation eines Kontrastmittels selbst eine gewisse Zeitspanne in Anspruch nimmt, definiert der Zeitpunkt TP0 vorzugsweise den Zeitpunkt, zu dem die Applikation abgeschlossen ist, Kontrastmittel also vollständig in das Untersuchungsobjekt eingebracht ist. Zum Zeitpunkt TP1 erreicht die Signalintensität des Kontrastmittels in den Leberarterien (A) ihr Maximum. Zum Zeitpunkt TP2 schneiden sich die Kurven der Signalintensitäten bei den Leberarterien (A) und den Lebervenen (V). Zum Zeitpunkt TP3 durchläuft die Signalintensität des Kontrastmittels in den Lebervenen (V) ihr Maximum. Zum Zeitpunkt TP4 schneiden sich die Kurven der Signalintensitäten bei den Leberarterien (A) und den gesunden Leberzellen (L). Zum Zeitpunkt TP5 sind die Konzentrationen in den Leberarterien (A) und den Lebervenen (V) auf ein Niveau gesunken, bei dem sie keine messbare Kontrastverstärkung mehr verursachen.

Während der kontrastverstärkten magnetresonanztomografischen Untersuchung werden mehrere magnetresonanztomografische Aufnahmen von der Leber des Patienten oder von einem Teil der Leber des Patienten erzeugt. Solche magnetresonanztomografischen Aufnahmen werden in dieser Beschreibung als Repräsentationen bezeichnet. Sie repräsentieren die Leber oder einen Teil der Leber des Patienten vor und/oder nach der Applikation eines Kontrastmittels. Dabei kann es sich um Repräsentationen im Ortsraum oder um Repräsentationen im Frequenzraum handeln.

In der Magnetresonanztomografie fallen die Rohdaten aufgrund des Messverfahrens üblicherweise als so genannte k-Raum-Daten an. Bei diesen k-Raum-Daten handelt es sich um eine Darstellung (Repräsentation) eines Untersuchungsbereichs im Frequenzraum. Solche k-Raum-Daten können mittels inverser Fouriertransformation in eine Repräsentation im Ortsraum umgewandelt werden. Umgekehrt können Repräsentationen im Ortsraum mittels Fouriertransformation in einer Repräsentation im Frequenzraum (auch als Ortsfrequenzraum oder Fourier-Raum oder Frequenzdomäne oder Fourier-Darstellung bezeichnet) umgewandelt werden.

Die in dieser Beschreibung beschriebenen Aktionen werden vorzugsweise mit Repräsentationen im Ortsraum durchgeführt.

Eine Repräsentation eines Untersuchungsbereichs (z.B. der Leber) im Ortsraum ist die für einen Menschen geläufigere Repräsentation; sie ist für den Menschen leichter erfassbar (verständlicher). Für eine solche Repräsentation im Ortsraum wird üblicherweise auch der Begriff "Bild" (engl.: *image*) verwendet.

Eine Repräsentation im Sinne der vorliegenden Erfindung kann eine zweidimensionale, dreidimensionale oder höherdimensionale Repräsentation sein. Üblicherweise liegen zweidimensionale Tomogramme (Schichtaufnahmen) vor oder es liegt ein Stapel von zweidimensionalen Tomogrammen (Schichtaufnahmen) vor.

Üblicherweise liegen die Repräsentationen in digitaler Form vor. Der Begriff "digital" bedeutet, dass die Repräsentationen von einer Maschine, in der Regel einem Computersystem, verarbeitet werden können. Unter "Verarbeitung" werden die bekannten Verfahren zur elektronischen Datenverarbeitung (EDV) verstanden. Ein Beispiel für ein übliches Format für eine digitale Repräsentation ist das DICOM-Format (DICOM: *Digital Imaging and Communications in Medicine*) - ein offener Standard zur Speicherung und zum Austausch von Informationen im medizinischen Bilddatenmanagement.

Aus Gründen der einfacheren Darstellung wird die Erfindung an einigen Stellen der vorliegenden Beschreibung anhand des Vorliegens von zweidimensionalen Bildern erläutert, ohne die Erfindung jedoch auf zweidimensionale Bilder beschränken zu wollen. Dem Fachmann ist klar, wie sich das jeweils Beschriebene auf Stapel zweidimensionaler Bilder, auf 3D-Aufnahmen oder auf Repräsentationen im Frequenzraum übertragen lässt (siehe hierzu z.B. M. Reisler, W. Semmler: Magnetresonanztomographie, Springer Verlag, 3. Auflage, 2002, ISBN: 978-3-642-63076-7).

Digitale Bilder können in verschiedenen Formaten vorliegen. Digitale Bilder können beispielsweise als Rastergrafiken kodiert sein. Rastergrafiken bestehen aus einer rasterförmigen Anordnung von so genannten Bildpunkten (Pixel) oder Volumenelementen (Voxel), denen jeweils eine Farbe bzw. ein Grauwert zugeordnet ist. Die Hauptmerkmale einer 2D-Rastergrafik sind daher die Bildgröße (Breite und Höhe gemessen in Pixel, umgangssprachlich auch Bildauflösung genannt) sowie die Farbtiefe. Einem Bildpunkt einer digitalen Bilddatei ist üblicherweise eine Farbe zugeordnet. Die für einen Bildpunkt verwendete Kodierung der Farbe definiert sich unter anderem über den Farbraum und die Farbtiefe. Der einfachste Fall ist ein Binärbild, bei dem ein Bildpunkt einen Schwarzweiß-Wert speichert. Bei einem Bild, dessen Farbe über den so genannten RGB-Farbraum definiert ist (RGB steht für die Grundfarben Rot, Grün und Blau), besteht jeder Bildpunkt aus drei Subpixeln, einem Subpixel für die Farbe Rot, einem Subpixel für die Farbe Grün und einem Subpixel für die Farbe Blau. Die Farbe eines Bildpunktes ergibt sich durch die Überlagerung (additives Mischen) der Farbwerte der Subpixel. Der Farbwert eines Subpixels kann beispielsweise in 256 Farbnuancen unterteilt sein, die Tonwerte genannt werden und üblicherweise von 0 bis 255 reichen. Die Farbnuance "0" eines jeden Farbkanals ist die dunkelste. Haben alle drei Kanäle den Tonwert 0, erscheint der entsprechende Bildpunkt schwarz; haben alle drei Kanäle den Tonwert 255, erscheint der entsprechende Bildpunkt weiß. Bei der Ausführung der vorliegenden Erfindung werden digitale Bilder bestimmten Operationen unterworfen. Die Operationen betreffen dabei überwiegend die Bildpunkte, bzw. die Tonwerte der einzelnen Bildpunkte (Pixel oder Voxel). Es gibt eine Vielzahl an möglichen digitalen Bildformaten und Farbkodierungen. Vereinfachend wird in dieser Beschreibung davon ausgegangen, dass die vorliegenden Bilder Graustufen-Rastergrafiken mit einer spezifischen Zahl an Bildpunkten sind, wobei jedem Bildpunkt ein Tonwert zugeordnet ist, der den Grauwert des Bildes angibt. Diese Annahme soll jedoch in keiner Weise limitierend verstanden werden. Dem Fachmann der Bildverarbeitung ist klar, wie er die Lehre dieser Beschreibung auf Bilddateien, die in anderen Bildformaten vorliegen und/oder bei denen die Farbwerte anders kodiert sind, übertragen kann.

Während der kontrastverstärkten magnetresonanztomographischen Untersuchung wird eine Mehrzahl an Repräsentationen der Leber oder eines Teils der Leber eines Patienten erzeugt.

Die Mehrzahl an Repräsentationen umfasst mindestens eine Repräsentation, die die Leber oder den Teil der Leber während der portalvenösen Phase repräsentiert, und mindestens eine Repräsentation, die die Leber oder den Teil der Leber während der Übergangsphase repräsentiert.

Vorzugsweise wird ferner mindestens eine Repräsentation der Leber oder des Teils der Leber vor der Applikation des hepatobiliären Kontrastmittels (vor TP0) erzeugt (native Aufnahme) und/oder mindestens eine Repräsentation der Leber oder des Teils der Leber in der arteriellen Phase.

In einer bevorzugten Ausführungsform werden mindestens die folgenden Aufnahmen erzeugt:
- mindestens eine erste Repräsentation, wobei die mindestens eine erste Repräsentation die Leber oder einen Teil der Leber während der Zeitspanne von TP1 bis TP3 repräsentiert,
- mindestens eine zweite Repräsentation, wobei die mindestens eine zweite Repräsentation die Leber oder den Teil der Leber während der Zeitspanne von TP3 bis TP5 repräsentiert,
wobei die Zeitpunkte TP0 in Fig. 1 dargestellt und in der Beschreibung zur Fig. 1 erläutert sind.

In einer weiteren bevorzugten Ausführungsform werden mindestens die folgenden Aufnahmen erzeugt:
- mindestens eine erste Repräsentation, wobei die mindestens eine erste Repräsentation die Leber oder einen Teil der Leber eines Patienten während der Zeitspanne von TP0 bis TP3 repräsentiert,
- mindestens eine zweite Repräsentation, wobei die mindestens eine zweite Repräsentation die Leber oder den Teil der Leber des Patienten während der Zeitspanne von TP2 bis TP5 repräsentiert,
- mindestens eine dritte Repräsentation, wobei die mindestens eine dritte Repräsentation die Leber oder den Teil der Leber des Patienten während der Zeitspanne TP4 bis 5 Minuten nach TP0 repräsentiert,
wobei die Zeitpunkte TP0 in Fig. 1 dargestellt und in der Beschreibung zur Fig. 1 erläutert sind.

In einer besonders bevorzugten Ausführungsform werden mindestens die folgenden Aufnahmen erzeugt:
- mindestens eine erste Repräsentation, wobei die mindestens eine erste Repräsentation die Leber oder einen Teil der Leber eines Patienten während der Zeitspanne von TP0 bis TP1 repräsentiert, und
- mindestens eine zweite Repräsentation, wobei die mindestens eine zweite Repräsentation die Leber oder einen Teil der Leber eines Patienten während der Zeitspanne von TP1 bis TP2 repräsentiert, und
- mindestens eine dritte Repräsentation, wobei die mindestens eine dritte Repräsentation die Leber oder einen Teil der Leber eines Patienten während der Zeitspanne von TP2 bis TP3 repräsentiert, und
- mindestens eine vierte Repräsentation, wobei die mindestens eine vierte Repräsentation die Leber oder einen Teil der Leber eines Patienten während der Zeitspanne von TP3 bis TP4 repräsentiert, und
- mindestens eine fünfte Repräsentation, wobei die mindestens eine fünfte Repräsentation die Leber oder den Teil der Leber des Patienten während der Zeitspanne TP4 bis TP5 Sekunden nach TP0 repräsentiert, und/oder
- mindestens eine sechste Repräsentation, wobei die mindestens eine sechste Repräsentation die Leber oder den Teil der Leber des Patienten während der Zeitspanne TP5 bis 5 Minuten nach TP0 repräsentiert,
wobei die Zeitpunkte TP0 in Fig. 1 dargestellt und in der Beschreibung zur Fig. 1 erläutert sind.

Die erzeugten Repräsentationen werden dem erfindungsgemäßen Computersystem zugeführt, das konfiguriert ist, die Repräsentationen automatisiert zu analysieren.

Der Begriff "automatisiert" bedeutet ohne Zutun eines Menschen.

Bei der Analyse wird ein Bereich oder es werden mehrere Bereiche in der Leber des Patienten identifiziert, bei dem/denen es zu einem Auswaschen von Kontrastmittel in der portalvenösen Phase und/oder der Übergangsphase kommt.

Das Auswaschen kann auf verschiedene Weise identifiziert werden:
In einer Ausführungsform werden diejenigen Bereiche in der Leber identifiziert, bei denen Kontrastmittel in der portalvenösen Phase und/oder der Übergangsphase zu einer geringeren Kontrastverstärkung als in einem Referenzgewebe führt. Die geringere Kontrastverstärkung in der portalvenösen und/oder Übergangsphase wird auch als Hypoenhancement bezeichnet. Hypoenhancement ist die im Vergleich zu einem Referenzgewebe niedrigere Signalintensität. Als Referenzgewebe wird in dieser Ausführungsform ein Gewebe verwendet, das keine Hepatozyten umfasst. Erfindungsgemäß wird Muskelgewebe als Referenzgewebe verwendet.

Erfindungsgemäß wird ein hepatobiliäres Kontrastmittel verwendet. In einer weiteren Ausführungsform werden diejenigen Bereiche in der Leber identifiziert, bei denen die Kontrastverstärkung in der portalvenösen Phase und/oder der Übergangsphase schneller sinkt als in dem Referenzgewebe. Als Referenzgewebe wird auch in dieser Ausführungsform ein Gewebe verwendet, das keine Hepatozyten umfasst. Erfindungsgemäß wird Muskelgewebe als Referenzgewebe verwendet.

Es wird der zeitliche Gradient der Signalintensität ermittelt und es werden diejenigen Bereiche identifiziert, in denen der zeitliche Gradient der Signalintensität negativ ist (Abnahme der Signalintensität mit zunehmender Zeit) und in denen der Betrag des zeitlichen Gradienten größer ist als der Betrags des negativen zeitlichen Gradienten der Signalintensität in dem Referenzgewebe.

Erfindungsgemäß wird ein hepatobiliäres Kontrastmittel verwendet.

In einer weiteren Ausführungsform werden diejenigen Bereiche in der Leber identifiziert, bei denen der Betrag des Gradienten der abnehmenden Kontrastverstärkung in der portalvenösen Phase und/oder der Übergangsphase größer ist als der Gradient der zunehmenden Kontrastverstärkung in gesundem Lebergewebe. Als Referenzgewebe wird in dieser Ausführungsform das gesunde Lebergewebe verwendet. Erfindungsgemäß wird als Kontrastmittel ein hepatobiliäres Kontrastmittel verwendet, das selektiv von gesundem Lebergewebe aufgenommen wird und dort in der portalvenösen und/oder der Übergangsphase zu einer allmählich ansteigenden Signalintensität führt. Es liegt also in dem gesunden Lebergewebe ein positiver zeitlicher Gradient der Signalintensität in der portalvenösen Phase und/oder der Übergangsphase vor. Es werden diejenigen Bereiche identifiziert, bei denen die Signalintensität in der portalvenösen Phase und/oder der Übergangsphase abnimmt, wobei der Betrag der Abnahme (die absolute Geschwindigkeit der Abnahme) größer ist als der Betrag der Zunahme der Signalintensität im gesunden Lebergewebe.

Es ist denkbar, die genannten Ausführungsformen miteinander zu kombinieren. Es ist denkbar, dass diejenigen Bereiche in der Leber identifiziert werden, bei denen Kontrastmittel in der portalvenösen Phase und/oder der Übergangsphase zu einer geringeren Kontrastverstärkung als in einem ersten Referenzgewebe führt und bei denen die Kontrastverstärkung in der portalvenösen Phase und/oder der Übergangsphase schneller sinkt als in einem zweiten Referenzgewebe und/oder bei denen der Betrag des Gradienten der abnehmenden Kontrastverstärkung in der portalvenösen Phase und/oder der Übergangsphase größer ist als der Gradient der zunehmenden Kontrastverstärkung in gesundem Lebergewebe. Bei dem ersten und/oder dem zweiten Referenzgewebe kann es sich z.B. um Muskelgewebe oder um gesundes Lebergewebe handeln.

In einer besonders bevorzugten Ausführungsform wird die vorliegende Erfindung verwendet, um hepatozelluläre Karzinome zu identifizieren. In dieser Ausführungsform wird ein Bereich oder es werden mehrere Bereiche identifiziert, die sowohl ein Hyperenhancement in der artieriellen Phase als auch ein Auswaschen in der protalvenösen Phase und/oder der Übergangsphase zeigen.

Hyperenhancement liegt vor, wenn ein Bereich im Vergleich zu einem Referenzgewebe eine höhere Signalintensität aufweist (siehe z.B. M .Kim et al.: Identification of Arterial Hyperenhancement in CT and MRI in Patients with Hepatocellular Carcinoma: Value of Unenhanced Images, Korean Journal of Radiology 2019, 20(2), 236-245). Als Referenzgewebe zur Erkennung von Hyperenhancement in der artieriellen Phase wird vorzugsweise gesundes Lebergewebe verwendet. Erfindungsgemäß wird ein hepatobiliäres Kontrastmittel verwendet. Vorzugsweise umfasst die Ausführungsform zum Identifizieren eines hepatozelluläres Karzinoms die folgenden Schritte:
- Empfangen einer Mehrzahl an Repräsentationen der Leber oder eines Teils der Leber eines Patienten,
   ∘wobei die Repräsentationen das Ergebnis einer kontrastverstärkten magnetresonanztomographischen Untersuchung der Leber oder des Teils der Leber sind,
   ∘ wobei mindestens eine erste Repräsentation die Leber oder den Teil der Leber während einer arteriellen Phase repräsentiert, wobei mindestens eine zweite Repräsentation die Leber oder den Teil der Leber während einer portalvenösen Phase repräsentiert, wobei mindestens eine dritte Repräsentation die Leber oder den Teil der Leber während einer Übergangsphase repräsentiert,
- Analysieren der mindestens einen ersten, zweiten und dritten Repräsentation, wobei ein Bereich oder mehrere Bereiche der Leber identifiziert werden, bei denen Kontrastmittel in der arteriellen Phase zu einer höheren Kontrastverstärkung als in einem ersten Referenzgewebe führt und bei denen Kontrastmittel in der portalvenösen und/oder Übergangsphase zu einer geringeren Kontrastverstärkung als in einem zweiten Referenzgewebe führt,
- Ausgeben einer Repräsentation der Leber oder des Teils der Leber, wobei in der Repräsentation der identifizierte Bereich hervorgehoben ist oder die identifizierten Bereiche hervorgehoben sind.

Wie bereits beschrieben ist das erste Referenzgewebe vorzugsweise gesundes Lebergewebe und das zweite Referenzgewebe ist erfindungsgemäß Muskelgewebe.

In einer weiteren bevorzugten Ausführungsform umfasst das Verfahren zum Identifizieren eines hepatozelluläres Karzinoms die Schritte:
- Empfangen einer Mehrzahl an Repräsentationen der Leber oder eines Teils der Leber eines Patienten,
   ∘ wobei die Repräsentationen das Ergebnis einer kontrastverstärkten magnetresonanztomographischen Untersuchung der Leber oder des Teils der Leber sind,
   ∘ wobei mindestens eine erste Repräsentation die Leber oder den Teil der Leber während einer arteriellen Phase repräsentiert, wobei mindestens eine zweite Repräsentation die Leber oder den Teil der Leber während einer portalvenösen Phase repräsentiert, wobei mindestens eine dritte Repräsentation die Leber oder den Teil der Leber während einer Übergangsphase repräsentiert,
- Analysieren der empfangenen Repräsentation, wobei ein Bereich oder mehrere Bereiche der Leber identifiziert werden, die durch folgende Merkmale charakterisiert sind:
   ∘ Kontrastmittel führt in der arteriellen Phase zu einem größeren Signalanstieg als in einem ersten Referenzgewebe, und
   ∘ die Kontrastverstärkung in der portalvenösen Phase und/oder der Übergangsphase sinkt schneller als in einem zweiten Referenzgewebe, wobei das zweite Referenzgewebe keine Hepatozyten umfasst, und/oder
   ∘ der Betrag des Gradienten der abnehmenden Kontrastverstärkung in der portalvenösen Phase und/oder der Übergangsphase ist größer als der Betrag des Gradienten der zunehmenden Kontrastverstärkung in gesundem Lebergewebe.

Das erste Referenzgewebe ist vorzugsweise gesundes Lebergewebe und das zweite Referenzgewebe ist erfindungsgemäß Muskelgewebe.

In Fig. 2 (a) und Fig. 2 (b) sind die Begriffe Hyperenhancement und Hypoenhancement grafisch verdeutlicht. In Fig 2 (a) und Fig 2 (b) ist die Intensität *I* des bei der MRT-Untersuchung gemessenen Signals für zwei verschiedene Bereiche in der Leber eines Patienten als Funktion der Zeit *t* dargestellt. Die durchgezogenen Kurven, die mit dem Bezugszeichen HCC versehen sind, zeigen jeweils den dynamischen Verlauf der Intensität *I* eines hepatozellulären Karzinoms; die gestrichelten Kurven, die mit dem Bezugszeichen L versehen sind, zeigen den dynamischen Verlauf der Intensität *I* von gesundem Lebergewebe. Die Bezugszeichen AP, PVP und TP zeigen die arterielle Phase (AP), die portalvenöse Phase (PVP) und die Übergangsphase (TP) an. Der Zeitpunkt TP0 zeigt jeweils den Zeitpunkt des Abschlusses der Applikation eines hepatobiliären Kontrastmittels an. Nach der Applikation steigen die Intensitäten der MRT-Signale, die auf die hepatozellulären Karzinome zurückzuführen sind, stark an. Die Intensitäten der MRT-Signale, die auf gesundes Lebergewebe zurückzuführen sind, steigen ebenfalls an - jedoch weniger stark: es liegt Hyperenhancement beim HCC-Gewebe gegenüber dem gesunden Lebergewebe in der arteriellen Phase AP vor.

Nach Durchlaufen eines Maximus sinken die Intensitäten der MRT-Signale, die auf das Gewebe des hepatozellulären Karzinoms zurückzuführen sind, wieder ab; im Fall der Kurve in Fig. 2 (a) schneller als im Fall der Kurve in Fig. 2 (b). Die Intensitäten der MRT-Signale, die auf gesundes Lebergewebe zurückzuführen sind, steigen auch nach Ablauf der arteriellen Phase AP weiter an; jedoch langsamer als in der arteriellen Phase: dies zeigt die Aufnahme von Kontrastmittel in den Leberzellen an.

Die durchgezogene Kurve (HCC) sinkt in Fig. 2 (a) in der portalvenösen Phase PVP unter die gestrichelte Kurve (L): die Signalverstärkung im hepatozellulären Karzinom ist geringer als die Signalverstärkung im gesunden Lebergewebe. Es liegt Hypoenhancement beim HCC-Gewebe gegenüber dem gesunden Lebergewebe in der portalvenösen Phase vor.

Im Fall des hepatozellulären Karzinoms in Fig. 2 (b) tritt das Hypoenhancement erst später ein. Die durchgezogene Linie in Fig. 2 (b) fällt erst nach Ablauf der portalvenösen Phase unter die gestrichelte Linie in Fig. 2 (b). Hypoenhancement tritt erst nach Ablauf der portalvenösen Phase ein.

Erfindungsgemäß wird dann das Vorliegen eines hepatozellulären Karzinoms angezeigt, wenn bei der Läsion ein Hyperenhancement gegenüber einem ersten Referenzgewebe in der arteriellen Phase vorliegt, und wenn bei der Läsion Hypoenhancement gegenüber einem zweiten Referenzgewebe in der portalvenösen Phase oder in der Übergangsphase vorliegt oder wenn die Kontrastverstärkung in der portalvenösen Phase und/oder der Übergangsphase schneller sinkt als in einem zweiten Referenzgewebe, wobei das zweite Referenzgewebe keine Hepatozyten umfasst, und/oder der Betrag des Gradienten der abnehmenden Kontrastverstärkung in der portalvenösen Phase und/oder der Übergangsphase größer ist als der Gradient der zunehmenden Kontrastverstärkung in gesundem Lebergewebe.

Zur Beurteilung, ob Hyperenhancement und/oder Hypoenhancement vorliegen, können die Grauwerte der Pixel oder Voxel der Ortsraum-Repräsentationen (der 2D-Bilder oder 3D-Bilder) von Läsionen und Referenzgewebe(n) analysiert werden.

Um über einen Bereich eine Aussage treffen zu können, ob dieser Bereich ein Hyperenhancement in einer Zeitspanne und ein Hypoenhancement in einer anderen Zeitspanne aufweist, muss der Bereich in den Repräsentationen, die den Bereich in den verschiedenen Zeitspannen repräsentieren, eindeutig identifiziert und wiedergefunden werden. Mit anderen Worten: die Beurteilung, ob Hyperenhancement in einem Bereich vorliegt, erfolgt auf Basis von mindestens einer ersten Repräsentation, die den Bereich nach der Applikation eines Kontrastmittels in der arteriellen Phase repräsentiert; die Beurteilung, ob Hypoenhancement in einem Bereich vorliegt, erfolgt auf Basis von mindestens einer zweiten Repräsentation, die den Bereich nach der Applikation eines Kontrastmittels in der portalvenösen Phase und/oder der Übergangsphase repräsentiert; der Bereich muss also sowohl in der ersten als auch in der zweiten Repräsentation eindeutig bestimmbar sein und es muss sich in der mindestens einen ersten und in der mindestens einen zweiten Repräsentation um denselben Bereich handeln.

Dazu können die erzeugten Repräsentationen einer Bildregistrierung unterworfen werden. Die Bildregistrierung (auch "Co-Registrierung" genannt) ist ein Prozess in der digitalen Bildverarbeitung und dient dazu, zwei oder mehrere Bilder derselben Szene, oder zumindest ähnlicher Szenen, bestmöglich miteinander in Übereinstimmung zu bringen. Dabei wird eines der Bilder als Referenzbild festgelegt, die anderen werden Objektbilder genannt. Um diese Objektbilder optimal an das Referenzbild anzupassen, wird eine ausgleichende Transformation berechnet. Die zu registrierenden Bilder unterscheiden sich voneinander, weil sie von unterschiedlichen Positionen, zu unterschiedlichen Zeitpunkten oder mit unterschiedlichen Sensoren aufgenommen wurden.

Im Fall der Repräsentationen der vorliegenden Erfindung wurden sie zu unterschiedlichen Zeitpunkten aufgenommen.

Das Ziel der Bildregistrierung ist also, diejenige Transformation zu finden, die ein gegebenes Objektbild bestmöglich mit dem Referenzbild in Übereinstimmung bringt. Das Ziel ist, dass nach Möglichkeit jedes Pixel/Voxel eines Bildes denselben Bereich im Körper eines Patienten repräsentiert wie das Pixel/Voxel eines anderen (co-registrierten) Bildes mit denselben Koordinaten.

Verfahren zur Bildregistrierung sind im Stand der Technik beschrieben (siehe z.B.: E.H. Seeley et al.: Co-registration of multi-modality imaging allows for comprehensive analysis of tumor-induced bone disease, Bone 2014, 61, 208-216; C. Bhushan et al.: Co-registration and distortion correction of diffusion and anatomical images based on inverse contrast normalization, Neuroimage 2015, 15, 115: 269-80; US20200214619; US20090135191; EP3639272A).

Die Co-Registrierung kann für jede gesamte Repräsentation (das gesamte Bild mit allen in dem Bild erfassten anatomischen Merkmalen) erfolgen. Es ist auch denkbar, die Co-Registrierung auf die Läsionen zu beschränken, d.h. die einzelnen Repräsentationen durch Transformation so zu verändern, dass zumindest die Läsionen in den einzelnen Repräsentationen durch die korrespondierende Pixel/Voxel repräsentiert werden (wobei korrespondierende Pixel/Voxel dieselben Koordinaten haben).

Es ist ferner denkbar, jede Repräsentation einem Segmentierungsverfahren zu unterziehen, das Läsionen in den Repräsentationen erkennt und diese als solche markiert.

Verfahren zur Erkennung und Segmentierung von Läsionen sind im Stand der Technik beschrieben (siehe z.B.: C. Krishnamurthy et al.: Snake-based liver lesion segmentation, 6th IEEE Southwest Symposium on Image Analysis and Interpretation 2004 pp. 187-191, doi: 10.1109/IAI.2004.1300971; F.-A. Maayan et al.: GAN-based synthetic medical image augmentation for increased CNN performance in liver lesion classification, Neurocomputing 2018, 321, 321-331; WO2005/106773; EP3629898A; WO2012/040410).

Der Bereich oder die Bereiche in einer Repräsentation, der/die Referenzgewebe repräsentieren, kann/können durch einen Radiologen oder automatisiert festgelegt/bestimmt werden.

Es ist also denkbar, dass ein Radiologe in den Repräsentationen der Leber oder eines Teils der Leber, einen oder mehrere Bereiche markiert, die als Referenzbereich(e) fungieren.

Für eine automatisierte Bestimmung eines oder mehrerer Referenzbereiche können auch Segmentierungsverfahren verwendet werden (siehe z.B. WO2020/144134), die beispielsweise Muskelgewebe und/oder Lebergewebe in den Repräsentationen erkennen und einen Referenzbereich festlegen, der das Muskelgewebe und/oder Lebergewebe repräsentiert.

Gemäß der Lehre der vorliegenden Erfindung liegt Hyperenhancement für einen Bereich (eine Läsion) vor, wenn die Grauwerte der Pixel/Voxel, die den Bereich repräsentieren, innerhalb der arteriellen Phase signifikant oberhalb der Grauwerte der Pixel/Voxel, die Referenzgewebe repräsentieren, liegen.

Hypoenhancement liegt für einen Bereich (eine Läsion) vor, wenn die Grauwerte der Pixel/Voxel, die den Bereich repräsentieren, innerhalb der arteriellen Phase signifikant oberhalb der Grauwerte der Pixel/Voxel, die das Referenzgewebe repräsentieren, liegen.

Der Begriff "signifikant" bedeutet, dass es sich bei einem höheren oder niedrigeren Grauwert um ein Messergebnis handelt, das außerhalb der Fehlergrenzen des Messsystems liegt.

Prinzipiell reicht es aus, zur Erkennung von Hyperenhancement den Grauwert eines Pixels/Voxels, das den Bereich repräsentiert, mit dem Grauwert eines Pixels/Voxels, das ein Referenzgewebe repräsentiert, zu vergleichen; ist der Grauwert des Pixels/Voxels des Bereichs größer als der Grauwert des Pixels/Voxels des Referenzbereichs (höhere Signalverstärkung), liegt Hyperenhancement vor; ansonsten nicht. Analog reicht es prinzipiell aus, zur Erkennung von Hypoenhancement den Grauwert eines Pixels/Voxels, das den Bereich repräsentiert, mit dem Grauwert eines Pixels/Voxels, das ein Referenzgewebe repräsentiert, zu vergleichen; ist der Grauwert des Pixels/Voxels des Bereichs kleiner als der Grauwert des Pixels/Voxels des Referenzbereichs (geringere Signalverstärkung), liegt Hypoenhancement vor; ansonsten nicht.

Vorzugsweise werden mehrere Pixel/Voxel des Bereichs als auch des Referenzbereichs ausgewertet. Vorzugsweise definieren die mehreren Pixel/Voxel einen zusammenhängenden Bereich in einer oder in mehreren Repräsentationen; mit anderen Worten: vorzugsweise repräsentieren die mehreren Pixel/Voxel einen zusammenhängenden Bereich im Körper des Patienten. Werden mehrere Pixel/Voxel verwendet, so kann ein Mittelwert des Grauwerts (oder eines anderen Werts, der die Signalintensität angibt) berechnet werden (z.B. der arithmetische Mittelwert). Der Vergleich zwischen einem Bereich und einem Referenzbereich erfolgt dann auf Basis der jeweiligen Mittelwerte. Anstelle oder zusätzlich zu einer Mittelung über mehrere örtlich nebeneinander liegende Pixel/Voxel kann auch eine Mittelung über mehrere Pixel/Voxel von zeitlich aufeinander folgenden Repräsentationen vorgenommen werden.

In einer bevorzugten Ausführungsform werden neben den Signalintensitäten auch Gradienten der Signalintensitäten ermittelt. Ein solcher Gradient kann für einen Bereich beispielsweise aus zwei Repräsentationen gewonnen werden, die den Bereich in einem zeitlichen Abstand zueinander repräsentieren. Der zeitliche Abstand kann beispielsweise im Bereich von 1 Sekunde bis 30 Sekunden liegen. Je kürzer der zeitliche Abstand ist, desto höher ist die Genauigkeit, mit der sich Änderungen (Gradienten) der Signalintensität bestimmen lassen. Vorzugsweise werden zur Bestimmung von Gradienten in einer Phase eine Zahl von 2 bis 5 Repräsentationen erzeugt, die die Leber oder einen Teil der Leber während der Phase (arterielle Phase, portalvenöse Phase, Übergangsphase) repräsentieren. Nimmt die Signalintensität für einen Bereich von einer Repräsentation zu der zeitlich nachfolgenden Repräsentation zu, so ist der Gradient positiv; nimmt die Signalintensität hingegen ab, ist er negativ. Die Größe des Gradienten gibt Auskunft darüber, wie stark (schnell) die Signalintensitätszunahme oder die Signalintensitätsabnahme ist. Durch die Bestimmung eines oder mehrerer Gradienten im Anschluss an die arterielle Phase kann beispielsweise ermittelt werden, wie schnell die Auswaschung von Kontrastmittel in der portalvenösen und/oder der Übergangsphase für einen Bereich ist. Diese Information kann zum Beispiel verwendet werden, um einen oder mehrere Zeitpunkte für die Aufnahme einer oder mehrerer zweiter (oder weiterer) Repräsentationen festzulegen.

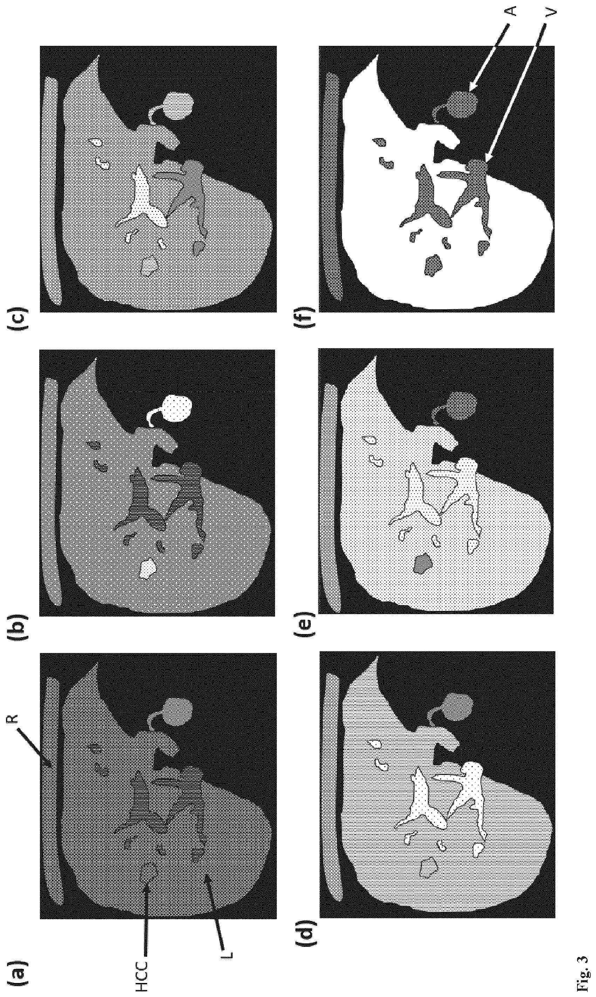

Figur 3 zeigt beispielhaft und schematisch Repräsentationen der Leber eines Patienten. In den Figuren 3 (a), 3 (b), 3 (c), 3 (d), 3 (e) und 3 (f) ist stets derselbe Querschnitt durch die Leber zu unterschiedlichen Zeitpunkten dargestellt. Neben der Leber ist auch Referenzgewebe (R) dargestellt; dabei kann es sich beispielsweise um Muskelgewebe handeln. Die in den Figuren 3 (a) und 3 (f) eingezeichneten Bezugszeichen gelten für alle Figuren 3 (a), 3 (b), 3 (c), 3 (d), 3 (e) und 3 (f); sie sind lediglich der besseren Übersicht halber jeweils nur einmal eingezeichnet.

Fig. 3 (a) zeigt den Querschnitt durch die Leber vor der intravenösen Applikation eines hepatobiliären Kontrastmittels (native Repräsentation). Zu einem Zeitpunkt, der zwischen den Zeitpunkten liegt, die durch die Figuren 3 (a) und 3 (b) dargestellt werden, wurde ein hepatobiliäres Kontrastmittel intravenös (z.B. in eine Armvene) als Bolus verabreicht. Dieses erreicht in Fig. 3 (b) über die Leberarterie (A) die Leber. Dementsprechend wird die Leberarterie (A) signalverstärkt dargestellt (arterielle Phase). Eine Läsion, die hauptsächlich über Arterien mit Blut versorgt wird, hebt sich ebenfalls als hellerer (signalverstärkter) Bereich vor dem gesunden Leberzellengewebe (L) und dem Referenzgewebe (R) ab. Zu dem Zeitpunkt, der in Figur 3 (c) dargestellt ist, erreicht das Kontrastmittel die Leber über die Venen (V). In Figur 3 (d) heben sich die venösen Blutgefäße (V) als helle (signalverstärkte) Bereiche von dem Lebergewebe (L) und dem Referenzgewebe (R) ab (portalvenöse Phase). Gleichzeitig steigt die Signalintensität in den gesunden Leberzellen (L), die hauptsächlich über die Venen mit Kontrastmittel versorgt werden, kontinuierlich an (Fig. 3 (c) → 3 (d) → 3 (e) → 3 (f)). In der hepatobiliären Phase, die in Fig. 3 (f) dargestellt ist, sind die Leberzellen (L) signalverstärkt dargestellt; die Blutgefäße, das Referenzgewebe und die Läsion weisen kein Kontrastmittel mehr auf und sind entsprechend dunkel dargestellt.

Es stellt sich die Frage, ob es sich bei der Läsion (HCC) in Fig. 3 um ein hepatozelluläres Karzinom handelt.

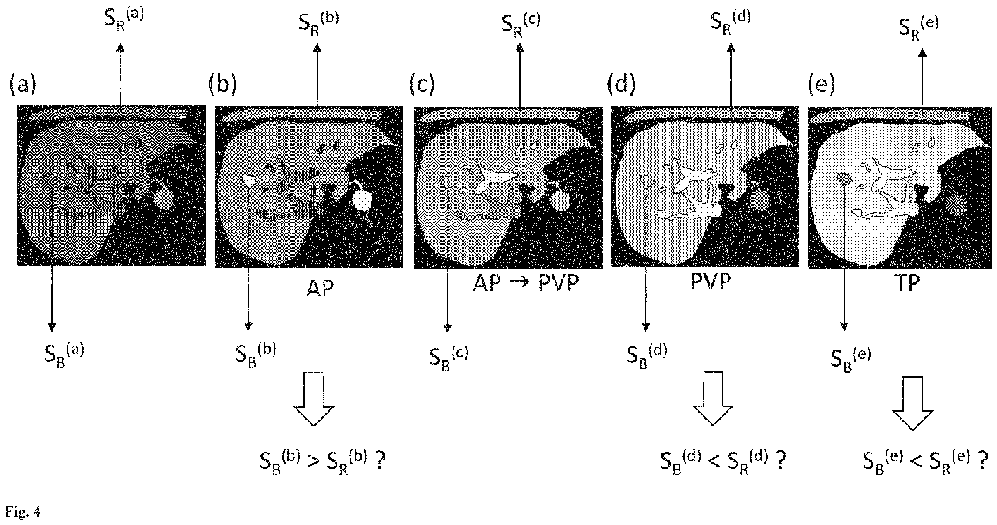

In Fig. 4 sind die Repräsentationen, die in Fig. 3 (a), 3 (b), 3 (c), 3 (d) und 3 (e) gezeigt sind, noch einmal verkleinert dargestellt. Sie tragen die Bezugszeichen (a), (b), (c), (d) und (e).

Für den Bereich in den Repräsentationen, der die Läsion repräsentiert, kann eine Signalintensität S_{B} ermittelt werden; für die Repräsentation (a) die Signalintensität S_{B}^{(a)}, für die Repräsentation (b) die Signalintensität S_{B}^{(b)}, für die Repräsentation (c) die Signalintensität S_{B}^{(c)}, für die Repräsentation (d) die Signalintensität S_{B}^{(d)} und für die Repräsentation (e) die Signalintensität S_{B}^{(e)}. Bei den Signalintensitäten kann es sich beispielsweise um die Grauwerte oder Farbwerte von Pixeln/Voxeln handeln, die den Bereich repräsentieren.

Analog kann für den Bereich in den Repräsentationen, der das Referenzgewebe repräsentiert, eine Signalintensität S_{R} ermittelt werden; für die Repräsentation (a) die Signalintensität S_{R}^{(a)}, für die Repräsentation (b) die Signalintensität S_{R}^{(b)}, für die Repräsentation (c) die Signalintensität S_{R}^{(c)}, für die Repräsentation (d) die Signalintensität S_{R}^{(d)} und für die Repräsentation (e) die Signalintensität S_{R}^{(e)}. Bei den Signalintensitäten kann es sich beispielsweise um die Grauwerte oder Farbwerte von Pixeln/Voxeln handeln, die den Bereich repräsentieren.

Um die Frage zu klären, ob es sich bei der Läsion um ein hepatozelluläres Karzinom handelt, werden Signalintensitäten in dem Bereich, der die Läsion repräsentiert, mit Signalintensitäten in dem Bereich, die das Referenzgewebe repräsentiert, verglichen, und zwar für mindestens eine Repräsentation während der arteriellen Phase AP und mindestens eine Repräsentation während der portalvenösen Phase PVP und/oder mindestens eine Repräsentation während der Übergangsphase TP. Die Repräsentation (b) repräsentiert die arterielle Phase AP. Es wird geprüft, ob die Signalintensität S_{B}^{(b)} in dem Bereich, der die Läsion repräsentiert, größer als die Signalintensität S_{R}^{(b)} in dem Bereich, der das Referenzgewebe repräsentiert, ist. Falls ja, liegt ein erstes Indiz dafür vor, dass es sich bei der Läsion um ein hepatozelluläres Karzinom handelt. Falls nein, müssen keine weiteren Signalintensitäten geprüft werden; ein hepatozelluläres Karzinom kann ausgeschlossen werden.

Die Repräsentation (d) repräsentiert die portalvenöse Phase PVP. Es wird geprüft, ob die Signalintensität S_{B}^{(d)} in dem Bereich, der die Läsion repräsentiert, kleiner als die Signalintensität S_{R}^{(d)} in dem Bereich, der das Referenzgewebe repräsentiert, ist. Falls ja, liegt ein zweites Indiz dafür vor, dass es sich bei der Läsion um ein hepatozelluläres Karzinom handelt. Falls das erste und das zweite Indiz vorliegen, wird die Läsion erfindungsgemäß als hepatozelluläres Karzinom angezeigt. Falls nur das erste aber nicht das zweite Indiz vorliegt, wird die Übergangsphase TP betrachtet. Die Repräsentation (e) repräsentiert die Übergangsphase TP. Es wird geprüft, ob die Signalintensität S_{B}^{(e)} in dem Bereich, der die Läsion repräsentiert, kleiner als die Signalintensität S_{R}^{(e)} in dem Bereich, der das Referenzgewebe repräsentiert, ist. Falls S_{B}^{(e)} kleiner als S_{R}^{(e)} ist und auch das erste Indiz vorliegt, wird erfindungsgemäß angezeigt, dass es sich bei der Läsion um ein hepatozelluläres Karzinom handelt.

Wurde bei einer Läsion erfindungsgemäß ermittelt, dass es sich dabei um ein hepatozelluläres Karzinom handelt, kann eine Mitteilung ausgegeben werden, dass Indizien für ein hepatozelluläres Karzinom vorliegen. Vorzugsweise wird eine Repräsentation der Leber oder eines Teils der Leber des Patienten ausgegeben, in der die Läsion gekennzeichnet ist, bei der Indizien für ein hepatozelluläres Karzinom vorliegen. Die entsprechende Läsion kann beispielsweise farbig markiert sein.

Die Erfindung kann mit Hilfe eines Computersystems ausgeführt werden.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem erfolgen über Eingabemittel wie beispielsweise eine Tastatur, eine Maus, ein Mikrofon, ein berührungsempfindliches Display und/oder dergleichen.

Ausgaben können auf einem Monitor, auf einem Drucker oder einem Datenspeicher erfolgen.

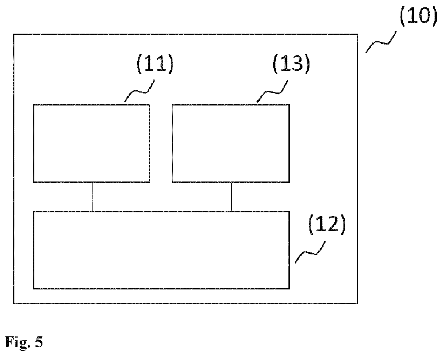

Figur 5 zeigt schematisch und beispielhaft eine Ausführungsform des erfindungsgemäßen Computersystems. Das Computersystem (10) umfasst eine Empfangseinheit (11), eine Steuer- und Recheneinheit (12) und eine Ausgabeeinheit (13).

Das erfindungsgemäße Computersystem (10) ist konfiguriert, Repräsentationen einer Leber oder eines Teils der Leber eines Patienten zu empfangen und einen oder mehrere Bereiche in den Repräsentationen zu identifizieren, die auf ein hepatozelluläres Karzinom hindeuten.

Die Steuer- und Recheneinheit (12) dient der Steuerung der Empfangseinheit (11) und der Ausgabeeinheit (13), der Koordinierung der Daten- und Signalflüsse zwischen den verschiedenen Einheiten, der Prozessierung von Repräsentationen und der Ermittlung dem Vergleich von Signalintensitäten. Es ist denkbar, dass mehrere Steuer- und Recheneinheiten vorhanden sind.

Die Empfangseinheit (11) dient zum Empfang der Repräsentationen. Die Repräsentationen können beispielsweise von einem Magnetresonanztomographen übermittelt werden oder aus einem Datenspeicher ausgelesen werden. Der Magnetresonanztomograph kann ein Bestandteil des erfindungsgemäßen Computersystems sein. Denkbar ist aber auch, dass das erfindungsgemäße Computersystem ein Bestandteil eines Magnetresonanztomographen ist. Die Übermittlung von Repräsentationen kann über eine Netzwerkverbindung oder eine direkte Verbindung erfolgen. Die Übermittlung von Repräsentationen kann über eine Funkverbindung (WLAN, Bluetooth, Mobilfunk und/oder dergleichen) und/oder kabelgebunden erfolgen. Es ist denkbar, dass mehrere Empfangseinheiten vorhanden sind. Auch der Datenspeicher kann ein Bestandteil des erfindungsgemäßen Computersystems sein oder mit diesem z.B. über ein Netzwerk verbunden sein. Es ist denkbar, dass mehrere Datenspeicher vorhanden sind.

Von der Empfangseinheit werden die Repräsentationen ggf. weitere Daten (wie beispielsweise Informationen zum Untersuchungsobjekt, Aufnahmeparameter und/oder dergleichen) empfangen und an die Steuer- und Recheneinheit übermittelt.

Die Steuer- und Recheneinheit ist konfiguriert, anhand der empfangenen Daten Bereiche zu identifizieren, die auf ein hepatozelluläres Karzinom hindeuten.

Über die Ausgabeeinheit (13) können die Ergebnisse der Analyse angezeigt werden (zum Beispiel auf einem Monitor), ausgegeben werden (z.B. über einen Drucker) oder in einem Datenspeicher gespeichert werden. Es ist denkbar, dass mehrere Ausgabeeinheiten vorhanden sind.

## Patentansprüche

1. Computer-implementiertes Verfahren umfassend die Schritte:
- Empfangen einer Mehrzahl an Repräsentationen,
∘ wobei die Mehrzahl an Repräsentationen
▪ eine Leber eines Patienten oder einen Teil der Leber des Patienten, und
▪ Referenzgewebe (R) des Patienten
repräsentiert,
∘ wobei mindestens eine Repräsentation der Mehrzahl an Repräsentationen
▪ die Leber oder den Teil der Leber, und
▪ das Referenzgewebe (R)
während einer portalvenösen Phase (PVP) einer dynamischen kontrastverstärkten magnetresonanztomographischen Untersuchung repräsentiert, und
∘ wobei mindestens eine Repräsentation der Mehrzahl an Repräsentationen
▪ die Leber oder den Teil der Leber, und
▪ das Referenzgewebe (R)
während einer Übergangsphase (TP) der dynamischen kontrastverstärkten magnetresonanztomographischen Untersuchung repräsentiert,
wobei es sich bei dem Kontrastmittel um ein hepatobiliäres Kontrastmittel handelt, wobei es sich bei dem Referenzgewebe (R) um Muskelgewebe handelt,
- Analysieren der Repräsentationen und dabei Identifizieren eines oder mehrerer Bereiche in der Leber,
∘ bei dem/denen Kontrastmittel in der portalvenösen Phase (PVP) und/oder der Übergangsphase (TP) zu einer geringeren Kontrastverstärkung als in dem Referenzgewebe (R) führt, und/oder
∘ bei dem/denen die Kontrastverstärkung in der portalvenösen Phase (PVP) und/oder der Übergangsphase (TP) schneller sinkt als in dem Referenzgewebe (R), wobei das Referenzgewebe keine Hepatozyten (R) umfasst, und/oder
∘ bei dem/denen der Betrag des Gradienten der abnehmenden Kontrastverstärkung in der portalvenösen Phase (PVP) und/oder der Übergangsphase (TP) größer ist als der Betrag des Gradienten der zunehmenden Kontrastverstärkung in gesundem Lebergewebe (L),
- Ausgeben einer Repräsentation der Leber oder des Teils der Leber, wobei in der Repräsentation der identifizierte Bereich hervorgehoben ist oder die identifizierten Bereiche hervorgehoben sind.

2. Verfahren gemäß Anspruch 1, wobei es sich bei dem hepatobiliären Kontrastmittel um das Dinatriumsalz der Gadoxetsäure handelt.

3. Verfahren gemäß einem der Ansprüche 1 bis 2, umfassend die Schritte:
- Empfangen der Mehrzahl an Repräsentationen,
∘ wobei mindestens zwei Repräsentationen der Mehrzahl an Repräsentationen
▪ die Leber oder den Teil der Leber, und
▪ das Referenzgewebe (R)
während der portalvenösen Phase (PVP) repräsentiert, und
∘ wobei mindestens zwei Repräsentationen der Mehrzahl an Repräsentationen
▪ die Leber oder den Teil der Leber, und
▪ das Referenzgewebe (R)
während der Übergangsphase (TP) repräsentieren,
- Analysieren der Mehrzahl an empfangenen Repräsentationen und dabei Identifizieren des einen oder der mehreren Bereiche in der Leber,
∘ bei dem/denen die Kontrastverstärkung in der portalvenösen Phase (PVP) und/oder der Übergangsphase (TP) schneller sinkt als in dem Referenzgewebe (R), wobei das Referenzgewebe (R) keine Hepatozyten umfasst, und/oder
∘ bei dem/denen der Betrag des Gradienten der abnehmenden Kontrastverstärkung in der portalvenösen Phase (PVP) und/oder der Übergangsphase (TP) größer ist als der Betrag des Gradienten der zunehmenden Kontrastverstärkung in gesundem Lebergewebe (L),
- Ausgeben der Repräsentation der Leber oder des Teils der Leber, wobei in der Repräsentation der identifizierte Bereich hervorgehoben ist oder die identifizierten Bereiche hervorgehoben sind.

4. Verfahren gemäß einem der Ansprüche 1 bis 3, ferner umfassend die Schritte:
- Empfangen mindestens einer Repräsentation,
∘ wobei die mindestens eine Repräsentation
▪ die Leber oder den Teil der Leber, und
▪ ein erstes und/oder zweites Referenzgewebe
während einer arteriellen Phase (AP) der dynamischen kontrastverstärkten magnetresonanztomographischen Untersuchung repräsentiert,
- Identifizieren eines oder mehrerer Bereiche in der Leber, wobei der Bereich / die Bereiche durch folgende Merkmale charakterisiert ist / sind:
∘ Kontrastmittel führt in der arteriellen Phase (AP) zu einer höheren Kontrastverstärkung als in dem ersten Referenzgewebe, wobei es sich bei dem ersten Referenzgewebe um gesundes Lebergewebe (L) oder Muskelgewebe handelt, und
∘ Kontrastmittel führt in der portalvenösen Phase (PVP) und/oder Übergangsphase (TP) zu einer geringeren Kontrastverstärkung als in dem zweiten Referenzgewebe führt, wobei es sich bei dem zweiten Referenzgewebe um Muskelgewebe handelt, und/oder
∘ die Kontrastverstärkung in der portalvenösen Phase (PVP) und/oder der Übergangsphase (TP) sinkt schneller als in dem zweiten Referenzgewebe, und/oder
∘ der Betrag des Gradienten der abnehmenden Kontrastverstärkung in der portalvenösen Phase (PVP) und/oder der Übergangsphase (TP) ist größer als der Betrag des Gradienten der zunehmenden Kontrastverstärkung in gesundem Lebergewebe (L),
- Ausgeben einer Repräsentation der Leber oder des Teils der Leber, wobei in der Repräsentation der identifizierte Bereich hervorgehoben ist oder die identifizierten Bereiche hervorgehoben sind.

5. Computersystem (10) umfassend
• eine Empfangseinheit (11),
• eine Steuer- und Recheneinheit (12) und
• eine Ausgabeeinheit (13),
- wobei die Steuer- und Recheneinheit (12) konfiguriert ist, die Empfangseinheit (10) zu veranlassen, eine Mehrzahl an Repräsentationen zu empfangen
∘ wobei die Mehrzahl an Repräsentationen
▪ eine Leber eines Patienten oder einen Teil der Leber des Patienten, und
▪ Referenzgewebe (R) des Patienten
repräsentiert,
∘ wobei mindestens eine Repräsentation der Mehrzahl an Repräsentationen
▪ die Leber oder den Teil der Leber, und
▪ das Referenzgewebe (R)
während einer portalvenösen Phase (PVP) einer dynamischen kontrastverstärkten magnetresonanztomographischen Untersuchung repräsentiert, und
∘ wobei mindestens eine Repräsentation der Mehrzahl an Repräsentationen
▪ die Leber oder den Teil der Leber, und
▪ das Referenzgewebe (R)
während einer Übergangsphase (TP) der dynamischen kontrastverstärkten magnetresonanztomographischen Untersuchung repräsentiert,
wobei es sich bei dem Kontrastmittel um ein hepatobiliäres Kontrastmittel handelt, wobei es sich bei dem Referenzgewebe (R) um Muskelgewebe handelt,
- wobei die Steuer- und Recheneinheit (12) konfiguriert ist, die Repräsentationen zu analysieren und dabei einen oder mehrere Bereiche in der Leber zu identifizieren,
∘bei dem/denen Kontrastmittel in der portalvenösen Phase (PVP) und/oder der Übergangsphase (TP) zu einer geringeren Kontrastverstärkung als in dem Referenzgewebe (R) führt, und/oder
∘ bei dem/denen die Kontrastverstärkung in der portalvenösen Phase (PVP) und/oder der Übergangsphase (TP) schneller sinkt als in dem Referenzgewebe (R), wobei das Referenzgewebe (R) keine Hepatozyten umfasst, und/oder
∘ bei dem/denen der Betrag des Gradienten der abnehmenden Kontrastverstärkung in der portalvenösen Phase (PVP) und/oder der Übergangsphase (TP) größer ist als der Betrag des Gradienten der zunehmenden Kontrastverstärkung in gesundem Lebergewebe (L),
- wobei die Steuer- und Recheneinheit (12) konfiguriert ist, die Ausgabeeinheit (13) zu veranlassen, eine Repräsentation der Leber oder des Teils der Leber auszugeben, wobei in der Repräsentation der identifizierte Bereich hervorgehoben ist oder die identifizierten Bereiche hervorgehoben sind.

6. Computerprogrammprodukt umfassend ein Computerprogramm, das in einen Arbeitsspeicher eines Computers geladen werden kann und dort den Computer dazu veranlasst, folgende Schritte ausführen:
- Empfangen einer Mehrzahl an Repräsentationen
∘ wobei die Mehrzahl an Repräsentationen
▪ eine Leber eines Patienten oder einen Teil der Leber des Patienten, und
▪ Referenzgewebe (R) des Patienten
repräsentiert,
∘ wobei mindestens eine Repräsentation der Mehrzahl an Repräsentationen
▪ die Leber oder den Teil der Leber, und
▪ das Referenzgewebe (R)
während einer portalvenösen Phase (PVP) einer dynamischen kontrastverstärkten magnetresonanztomographischen Untersuchung repräsentiert, und
∘ wobei mindestens eine Repräsentation der Mehrzahl an Repräsentationen
▪ die Leber oder den Teil der Leber, und
▪ das Referenzgewebe (R)
während einer Übergangsphase (TP) der dynamischen kontrastverstärkten magnetresonanztomographischen Untersuchung repräsentiert,
wobei es sich bei dem Kontrastmittel um ein hepatobiliäres Kontrastmittel handelt, wobei es sich bei dem Referenzgewebe (R) um Muskelgewebe handelt,
- Analysieren der Repräsentationen und dabei Identifizieren eines oder mehrerer Bereiche in der Leber,
∘ bei dem/denen Kontrastmittel in der portalvenösen Phase (PVP) und/oder der Übergangsphase (TP) zu einer geringeren Kontrastverstärkung als in dem Referenzgewebe (R) führt, und/oder
∘ bei dem/denen die Kontrastverstärkung in der portalvenösen Phase (PVP) und/oder der Übergangsphase (TP) schneller sinkt als in dem Referenzgewebe (R), wobei das Referenzgewebe (R) keine Hepatozyten umfasst, und/oder
∘ bei dem/denen der Betrag des Gradienten der abnehmenden Kontrastverstärkung in der portalvenösen Phase (PVP) und/oder der Übergangsphase (TP) größer ist als der Betrag des Gradienten der zunehmenden Kontrastverstärkung in gesundem Lebergewebe (L),
- Ausgeben einer Repräsentation der Leber oder des Teils der Leber, wobei in der Repräsentation der identifizierte Bereich hervorgehoben ist oder die identifizierten Bereiche hervorgehoben sind.

7. Verwendung eines hepatobiliären Kontrastmittels in einem dynamischen kontrastverstärkten magnetresonanztomographischen Untersuchungsverfahren, wobei das Untersuchungsverfahren die folgenden Schritte umfasst:
- Applizieren des Kontrastmittels in eine von einer Arterie entfernte Vene eines Patienten,
- Erzeugen einer Mehrzahl an Repräsentationen
∘ wobei die Mehrzahl an Repräsentationen
▪ die Leber des Patienten oder einen Teil der Leber des Patienten, und
▪ Referenzgewebe (R) des Patienten
repräsentiert,
∘ wobei mindestens eine Repräsentation der Mehrzahl an Repräsentationen
▪ die Leber oder den Teil der Leber, und
▪ das Referenzgewebe (R)
während einer portalvenösen Phase (PVP) des Untersuchungsverfahrens repräsentiert, und
∘ wobei mindestens eine Repräsentation der Mehrzahl an Repräsentationen
▪ die Leber oder den Teil der Leber, und
▪ das Referenzgewebe (R)
während einer Übergangsphase (TP) des Untersuchungsverfahrens repräsentiert,
wobei es sich bei dem Referenzgewebe (R) um Muskelgewebe handelt,
- Analysieren der Repräsentationen und dabei Identifizieren eines oder mehrerer Bereiche in der Leber,
∘ bei dem/denen Kontrastmittel in der portalvenösen Phase (PVP) und/oder der Übergangsphase (TP) zu einer geringeren Kontrastverstärkung als in dem Referenzgewebe (R) führt, und/oder
∘ bei dem/denen die Kontrastverstärkung in der portalvenösen Phase (PVP) und/oder der Übergangsphase (TP) schneller sinkt als in dem Referenzgewebe (R), wobei das Referenzgewebe (R) keine Hepatozyten umfasst, und/oder
∘ bei dem/denen der Betrag des Gradienten der abnehmenden Kontrastverstärkung in der portalvenösen Phase (PVP) und/oder der Übergangsphase (TP) größer ist als der Betrag des Gradienten der zunehmenden Kontrastverstärkung in gesundem Lebergewebe (L),
- Ausgeben einer Repräsentation der Leber oder des Teils der Leber, wobei in der Repräsentation der identifizierte Bereich hervorgehoben ist oder die identifizierten Bereiche hervorgehoben sind.

8. Kit umfassend ein Kontrastmittel und ein Computerprogrammprodukt gemäß Anspruch 6.

9. Kit gemäß Anspruch 8, wobei es sich bei dem Kontrastmittel um das Dinatriumsalz der Gadoxetsäure handelt.

## Claims

1. Computer-implemented method comprising the steps of:
- receiving a plurality of representations,
∘ wherein the plurality of representations represents
▪ a liver of a patient or part of the liver of the patient, and
▪ reference tissue (R) of the patient,
∘ wherein at least one representation of the plurality of representations represents
▪ the liver or the part of the liver, and
▪ the reference tissue (R)
during a portal venous phase (PVP) of a dynamic contrast-enhanced magnetic resonance imaging examination, and
∘ wherein at least one representation of the plurality of representations represents
▪ the liver or the part of the liver, and
▪ the reference tissue (R)
during a transitional phase (TP) of the dynamic contrast-enhanced magnetic resonance imaging examination,
wherein the contrast agent is a hepatobiliary contrast agent, wherein the reference tissue (R) is muscle tissue,
- analysing the representations and, in doing so, identifying one or more regions in the liver
∘ in which contrast agent leads in the portal venous phase (PVP) and/or the transitional phase (TP) to a lower contrast enhancement than in the reference tissue (R), and/or
∘ in which the contrast enhancement in the portal venous phase (PVP) and/or the transitional phase (TP) drops more rapidly than in the reference tissue (R), wherein the reference tissue does not comprise hepatocytes (R), and/or
∘ in which the absolute value of the gradient of the decreasing contrast enhancement in the portal venous phase (PVP) and/or the transitional phase (TP) is greater than the absolute value of the gradient of the increasing contrast enhancement in healthy liver tissue (L),
- outputting a representation of the liver or the part of the liver, wherein in the representation the identified region is highlighted or the identified regions are highlighted.

2. Method according to Claim 1, wherein the hepatobiliary contrast agent is the disodium salt of gadoxetic acid.

3. Method according to any of Claims 1 to 2, comprising the steps of:
- receiving the plurality of representations,
∘ wherein at least two representations of the plurality of representations represent
▪ the liver or the part of the liver, and
▪ the reference tissue (R)
during the portal venous phase (PVP), and
∘ wherein at least two representations of the plurality of representations represent
▪ the liver or the part of the liver, and
▪ the reference tissue (R)
during the transitional phase (TP),
- analysing the plurality of received representations and, in doing so, identifying the one region or the multiple regions in the liver
∘ in which the contrast enhancement in the portal venous phase (PVP) and/or the transitional phase (TP) drops more rapidly than in the reference tissue (R), wherein the reference tissue (R) does not comprise hepatocytes, and/or
∘ in which the absolute value of the gradient of the decreasing contrast enhancement in the portal venous phase (PVP) and/or the transitional phase (TP) is greater than the absolute value of the gradient of the increasing contrast enhancement in healthy liver tissue (L),
- outputting the representation of the liver or the part of the liver, wherein in the representation the identified region is highlighted or the identified regions are highlighted.

4. Method according to any of Claims 1 to 3, further comprising the steps of:
- receiving at least one representation,
∘ wherein the at least one representation represents
▪ the liver or the part of the liver, and
▪ a first and/or second reference tissue
during an arterial phase (AP) of the dynamic contrast-enhanced magnetic resonance imaging examination,
- identifying one or more regions in the liver, wherein the region/regions is/are **characterized by** the following features:
∘ contrast agent leads in the arterial phase (AP) to a higher contrast enhancement than in the first reference tissue, wherein the first reference tissue is healthy liver tissue (L) or muscle tissue, and
∘ contrast agent leads in the portal venous phase (PVP) and/or transitional phase (TP) to a lower contrast enhancement than in the second reference tissue, wherein the second reference tissue is muscle tissue, and/or
∘ the contrast enhancement in the portal venous phase (PVP) and/or the transitional phase (TP) drops more rapidly than in the second reference tissue, and/or
∘ the absolute value of the gradient of the decreasing contrast enhancement in the portal venous phase (PVP) and/or the transitional phase (TP) is greater than the absolute value of the gradient of the increasing contrast enhancement in healthy liver tissue (L),
- outputting a representation of the liver or the part of the liver, wherein in the representation the identified region is highlighted or the identified regions are highlighted.

5. Computer system (10) comprising
• a receiving unit (11),
• a control and calculation unit (12) and
• an output unit (13),
- wherein the control and calculation unit (12) is configured to cause the receiving unit (10) to receive a plurality of representations
∘ wherein the plurality of representations represents
▪ a liver of a patient or part of the liver of the patient, and
▪ reference tissue (R) of the patient,
o wherein at least one representation of the plurality of representations represents
▪ the liver or the part of the liver, and
▪ the reference tissue (R)
during a portal venous phase (PVP) of a dynamic contrast-enhanced magnetic resonance imaging examination, and
∘ wherein at least one representation of the plurality of representations represents
▪ the liver or the part of the liver, and
▪ the reference tissue (R)
during a transitional phase (TP) of the dynamic contrast-enhanced magnetic resonance imaging examination,
wherein the contrast agent is a hepatobiliary contrast agent, wherein the reference tissue (R) is muscle tissue,
- wherein the control and calculation unit (12) is configured to analyse the representations and, in doing so, identify one or more regions in the liver
∘ in which contrast agent leads in the portal venous phase (PVP) and/or the transitional phase (TP) to a lower contrast enhancement than in the reference tissue (R), and/or
∘ in which the contrast enhancement in the portal venous phase (PVP) and/or the transitional phase (TP) drops more rapidly than in the reference tissue (R), wherein the reference tissue (R) does not comprise hepatocytes, and/or
∘ in which the absolute value of the gradient of the decreasing contrast enhancement in the portal venous phase (PVP) and/or the transitional phase (TP) is greater than the absolute value of the gradient of the increasing contrast enhancement in healthy liver tissue (L),
- wherein the control and calculation unit (12) is configured to cause the output unit (13) to output a representation of the liver or the part of the liver, wherein in the representation the identified region is highlighted or the identified regions are highlighted.

6. Computer program product comprising a computer program which can be loaded into a working memory of a computer, where it causes the computer to execute the following steps:
- receiving a plurality of representations
∘ wherein the plurality of representations represents
▪ a liver of a patient or part of the liver of the patient, and
▪ reference tissue (R) of the patient,
o wherein at least one representation of the plurality of representations represents
▪ the liver or the part of the liver, and
▪ the reference tissue (R)
during a portal venous phase (PVP) of a dynamic contrast-enhanced magnetic resonance imaging examination, and
∘ wherein at least one representation of the plurality of representations represents
▪ the liver or the part of the liver, and
▪ the reference tissue (R)
during a transitional phase (TP) of the dynamic contrast-enhanced magnetic resonance imaging examination,
wherein the contrast agent is a hepatobiliary contrast agent, wherein the reference tissue (R) is muscle tissue,
- analysing the representations and, in doing so, identifying one or more regions in the liver
∘ in which contrast agent leads in the portal venous phase (PVP) and/or the transitional phase (TP) to a lower contrast enhancement than in the reference tissue (R), and/or
∘ in which the contrast enhancement in the portal venous phase (PVP) and/or the transitional phase (TP) drops more rapidly than in the reference tissue (R), wherein the reference tissue (R) does not comprise hepatocytes, and/or
∘ in which the absolute value of the gradient of the decreasing contrast enhancement in the portal venous phase (PVP) and/or the transitional phase (TP) is greater than the absolute value of the gradient of the increasing contrast enhancement in healthy liver tissue (L),
- outputting a representation of the liver or the part of the liver, wherein in the representation the identified region is highlighted or the identified regions are highlighted.

7. Use of a hepatobiliary contrast agent in a dynamic contrast-enhanced magnetic resonance imaging examination method, wherein the examination method comprises the following steps:
- administering the contrast agent into a vein of a patient remote from an artery,
- generating a plurality of representations
∘ wherein the plurality of representations represents
▪ the liver of the patient or part of the liver of the patient, and
▪ reference tissue (R) of the patient,
∘ wherein at least one representation of the plurality of representations represents
▪ the liver or the part of the liver, and
▪ the reference tissue (R)
during a portal venous phase (PVP) of the examination method, and
∘ wherein at least one representation of the plurality of representations represents
▪ the liver or the part of the liver, and
▪ the reference tissue (R)
during a transitional phase (TP) of the examination method,
wherein the reference tissue (R) is muscle tissue,
- analysing the representations and, in doing so, identifying one or more regions in the liver
∘ in which contrast agent leads in the portal venous phase (PVP) and/or the transitional phase (TP) to a lower contrast enhancement than in the reference tissue (R), and/or
∘ in which the contrast enhancement in the portal venous phase (PVP) and/or the transitional phase (TP) drops more rapidly than in the reference tissue (R), wherein the reference tissue (R) does not comprise hepatocytes, and/or
∘ in which the absolute value of the gradient of the decreasing contrast enhancement in the portal venous phase (PVP) and/or the transitional phase (TP) is greater than the absolute value of the gradient of the increasing contrast enhancement in healthy liver tissue (L),
- outputting a representation of the liver or the part of the liver, wherein in the representation the identified region is highlighted or the identified regions are highlighted.

8. Kit comprising a contrast agent and a computer program product according to Claim 6.

9. Kit according to Claim 8, wherein the contrast agent is the disodium salt of gadoxetic acid.

## Revendications

1. Procédé mis en œuvre par ordinateur, comprenant les étapes consistant à :
- recevoir une pluralité de représentations
∘ la pluralité de représentations représentant
▪ le foie d'un patient ou une partie du foie du patient, et
▪ un tissu de référence (R) du patient,
∘ au moins une représentation de la pluralité de représentations représentant
▪ le foie ou la partie du foie, et
▪ le tissu de référence (R)
pendant une phase portale veineuse (PVP) d'un examen par tomographie par résonance magnétique dynamique à contraste amélioré, et
∘ au moins une représentation de la pluralité de représentations représentant
▪ le foie ou la partie du foie, et
▪ le tissu de référence (R)
pendant une phase de transition (TP) de l'examen par tomographie par résonance magnétique dynamique à contraste amélioré,
l'agent de contraste étant un agent de contraste hépatobiliaire, le tissu de référence (R) étant un tissu musculaire,
- analyser les représentations et identifier ainsi une ou plusieurs zones dans le foie,
∘ dans laquelle/lesquelles l'agent de contraste conduit à un moindre rehaussement du contraste dans la phase portale veineuse (PVP) et/ou la phase de transition (TP) que dans le tissu de référence (R), et/ou
∘ dans laquelle/lesquelles le rehaussement du contraste dans la phase portale veineuse (PVP) et/ou la phase de transition (TP) diminue plus rapidement que dans le tissu de référence (R), le tissu de référence ne comprenant pas d'hépatocytes (R), et/ou
∘ dans laquelle/lesquelles la valeur absolue du gradient de la diminution du rehaussement du contraste dans la phase portale veineuse (PVP) et/ou la phase de transition (TP) est supérieure à la valeur absolue du gradient de l'augmentation du rehaussement du contraste dans le tissu hépatique sain (L),
- délivrer une représentation du foie ou de la partie du foie, la zone identifiée étant mise en évidence ou les zones identifiées étant mises en évidence dans la représentation.

2. Procédé selon la revendication 1, dans lequel l'agent de contraste hépatobiliaire est le sel disodique de l'acide gadoxétique.

3. Procédé selon l'une quelconque des revendications 1 à 2, comprenant les étapes consistant à :
- recevoir la pluralité de représentations
∘ au moins deux représentations de la pluralité de représentations représentant
▪ le foie ou la partie du foie, et
▪ le tissu de référence (R)
pendant la phase portale veineuse (PVP), et
∘ au moins deux représentations de la pluralité de représentations représentant
▪ le foie ou la partie du foie, et
▪ le tissu de référence (R)
pendant la phase de transition (TP),
- analyser la pluralité de représentations reçues et identifier ainsi la ou les zones dans le foie,
∘ dans laquelle/lesquelles le rehaussement du contraste dans la phase portale veineuse (PVP) et/ou la phase de transition (TP) diminue plus rapidement que dans le tissu de référence (R), le tissu de référence ne comprenant pas d'hépatocytes (R), et/ou
∘ dans laquelle/lesquelles la valeur absolue du gradient de la diminution du rehaussement du contraste dans la phase portale veineuse (PVP) et/ou la phase de transition (TP) est supérieure à la valeur absolue du gradient de l'augmentation du rehaussement du contraste dans le tissu hépatique sain (L),
- délivrer une représentation du foie ou de la partie du foie, la zone identifiée étant mise en évidence ou les zones identifiées étant mises en évidence dans la représentation.

4. Procédé selon l'une quelconque des revendications 1 à 3, comprenant en outre les étapes consistant à :
- recevoir au moins une représentation,
∘ ladite au moins une représentation représentant
▪ le foie ou la partie du foie, et
▪ un premier et/ou deuxième tissu de référence
pendant une phase artérielle (AP) de l'examen par tomographie par résonance magnétique dynamique à contraste amélioré,
- identifier une ou plusieurs zones dans le foie, la zone/les zones étant caractérisée(s) par les caractéristiques suivantes :
∘ l'agent de contraste conduit à un rehaussement du contraste plus élevé dans la phase artérielle (AP) que dans le premier tissu de référence, le premier tissu de référence étant un tissu hépatique sain (L) ou un tissu musculaire, et
∘ l'agent de contraste conduit à un moindre rehaussement du contraste dans la phase portale veineuse (PVP) et/ou la phase de transition (TP) que dans le deuxième tissu de référence, le deuxième tissu de référence étant un tissu musculaire, et/ou
∘ le rehaussement du contraste diminue plus rapidement dans la phase portale veineuse (PVP) et/ou la phase de transition (TP) que dans le deuxième tissu de référence, et/ou
o la valeur absolue du gradient de la diminution du rehaussement du contraste dans la phase portale veineuse (PVP) et/ou la phase de transition (TP) est supérieure à la valeur absolue du gradient de l'augmentation du rehaussement du contraste dans le tissu hépatique sain (L),
- délivrer une représentation du foie ou de la partie du foie, la zone identifiée étant mise en évidence ou les zones identifiées étant mises en évidence dans la représentation.

5. Système informatique (10) comprenant
• une unité de réception (11),
• une unité (12) de commande et de calcul, et
• une unité de sortie (13),
- l'unité (12) de commande et de calcul étant configurée pour amener l'unité de réception (10) à une pluralité de représentations,
∘ la pluralité de représentations représentant
▪ le foie d'un patient ou une partie du foie du patient, et
▪ un tissu de référence (R) du patient,
∘ au moins une représentation de la pluralité de représentations représentant
▪ le foie ou la partie du foie, et
▪ le tissu de référence (R)
pendant une phase portale veineuse (PVP) d'un examen par tomographie par résonance magnétique dynamique à contraste amélioré, et
∘ au moins une représentation de la pluralité de représentations représentant
▪ le foie ou la partie du foie, et
▪ le tissu de référence (R)
pendant une phase de transition (TP) de l'examen par tomographie par résonance magnétique dynamique à contraste amélioré,
l'agent de contraste étant un agent de contraste hépatobiliaire, le tissu de référence (R) étant un tissu musculaire,
- l'unité de commande et de calcul (12) étant configurée pour analyser les représentations et identifier ainsi une ou plusieurs zones dans le foie,
∘ dans laquelle/lesquelles l'agent de contraste conduit à un moindre rehaussement du contraste dans la phase portale veineuse (PVP) et/ou la phase de transition (TP) que dans le tissu de référence (R), et/ou
∘ dans laquelle/lesquelles le rehaussement du contraste dans la phase portale veineuse (PVP) et/ou la phase de transition (TP) diminue plus rapidement que dans le tissu de référence (R), le tissu de référence ne comprenant pas d'hépatocytes (R), et/ou
∘ dans laquelle/lesquelles la valeur absolue du gradient de la diminution du rehaussement du contraste dans la phase portale veineuse (PVP) et/ou la phase de transition (TP) est supérieure à la valeur absolue du gradient de l'augmentation du rehaussement du contraste dans le tissu hépatique sain (L),
- l'unité de commande et de calcul (12) étant configurée pour amener l'unité d'émission (13) à délivrer une représentation du foie ou de la partie du foie, la zone identifiée étant mise en évidence ou les zones identifiées étant mises en évidence dans la représentation.

6. Produit programme informatique, comprenant un programme informatique qui peut être chargé dans une mémoire vive d'un ordinateur et qui y amène l'ordinateur à exécuter les étapes suivantes :
- recevoir une pluralité de représentations
∘ la pluralité de représentations représentant
▪ le foie d'un patient ou une partie du foie du patient, et
▪ un tissu de référence (R) du patient,
∘ au moins une représentation de la pluralité de représentations représentant
▪ le foie ou la partie du foie, et
▪ le tissu de référence (R)
pendant une phase portale veineuse (PVP) d'un examen par tomographie par résonance magnétique dynamique à contraste amélioré, et
∘ au moins une représentation de la pluralité de représentations représentant
▪ le foie ou la partie du foie, et
▪ le tissu de référence (R)
pendant une phase de transition (TP) de l'examen par tomographie par résonance magnétique dynamique à contraste amélioré,
l'agent de contraste étant un agent de contraste hépatobiliaire, le tissu de référence (R) étant un tissu musculaire,
- analyser les représentations et identifier ainsi une ou plusieurs zones dans le foie,
∘ dans laquelle/lesquelles l'agent de contraste conduit à un moindre rehaussement du contraste dans la phase portale veineuse (PVP) et/ou la phase de transition (TP) que dans le tissu de référence (R), et/ou
∘ dans laquelle/lesquelles le rehaussement du contraste dans la phase portale veineuse (PVP) et/ou la phase de transition (TP) diminue plus rapidement que dans le tissu de référence (R), le tissu de référence ne comprenant pas d'hépatocytes (R), et/ou
∘ dans laquelle/lesquelles la valeur absolue du gradient de la diminution du rehaussement du contraste dans la phase portale veineuse (PVP) et/ou la phase de transition (TP) est supérieure à la valeur absolue du gradient de l'augmentation du rehaussement du contraste dans le tissu hépatique sain (L),
- délivrer une représentation du foie ou de la partie du foie, la zone identifiée étant mise en évidence ou les zones identifiées étant mises en évidence dans la représentation.

7. Utilisation d'un agent de contraste hépatobiliaire dans un procédé d'examen par tomographie par résonance magnétique dynamique à contraste amélioré, le procédé d'examen comprenant les étapes suivantes :
- appliquer l'agent de contraste dans une veine éloignée d'une artère d'un patient,
- générer une pluralité de représentations
∘ la pluralité de représentations représentant
▪ le foie d'un patient ou une partie du foie du patient, et
▪ un tissu de référence (R) du patient,
∘ au moins une représentation de la pluralité de représentations représentant
▪ le foie ou la partie du foie, et
▪ le tissu de référence (R)
pendant une phase portale veineuse (PVP) du procédé d'examen, et
∘ au moins une représentation de la pluralité de représentations représentant
▪ le foie ou la partie du foie, et
▪ le tissu de référence (R)
pendant une phase de transition (TP) du procédé d'examen, le tissu de référence (R) étant un tissu musculaire,
- analyser les représentations et identifier ainsi une ou plusieurs zones dans le foie,
∘ dans laquelle/lesquelles l'agent de contraste conduit à un moindre rehaussement du contraste dans la phase portale veineuse (PVP) et/ou la phase de transition (TP) que dans le tissu de référence (R), et/ou
∘ dans laquelle/lesquelles le rehaussement du contraste dans la phase portale veineuse (PVP) et/ou la phase de transition (TP) diminue plus rapidement que dans le tissu de référence (R), le tissu de référence ne comprenant pas d'hépatocytes (R), et/ou
∘ dans laquelle/lesquelles la valeur absolue du gradient de la diminution du rehaussement du contraste dans la phase portale veineuse (PVP) et/ou la phase de transition (TP) est supérieure à la valeur absolue du gradient de l'augmentation du rehaussement du contraste dans le tissu hépatique sain (L),
- délivrer une représentation du foie ou de la partie du foie, la zone identifiée étant mise en évidence ou les zones identifiées étant mises en évidence dans la représentation.

8. Kit comprenant un agent de contraste et un produit programme informatique selon la revendication 6.

9. Procédé selon la revendication 8, dans lequel l'agent de contraste est le sel disodique de l'acide gadoxétique.
